# EUROPÄISCHE PATENTANMELDUNG

(11) **EP 2 192 509 A1**
(43) Veröffentlichungstag der Anmeldung: **02.06.2010**
(21) Anmeldenummer: 08169431.7
(22) Anmeldetag: 19.11.2008
(51) Int. Cl.: G06F 19/00

(54) **Verfahren zur Anzeige von patientenbezogenen Diagnosen chronischer Krankheiten**

(71) Anmelder: CompuGroup Holding AG, 56070 Koblenz (DE)
(72) Erfinder: Gotthardt, Frank, 56337 Eitelborn (DE); Heimann, Dierk, Dr. med., 56070 Koblenz (DE)
(74) Vertreter: Richardt, Markus Albert

(57) **Zusammenfassung**

Die Erfindung betrifft ein Verfahren zur Anzeige von patientenbezogenen Diagnosen chronischer Krankheiten auf einer graphischen Benutzeroberfläche (100) eines Datenverarbeitungssystems, wobei die graphische Benutzeroberfläche (100) zumindest ein erstes (114) Anzeigefenster mit Patientendaten und ein zweites (110) Anzeigefenster mit Dauerdiagnosen aufweist.

## Beschreibung

Die Erfindung betrifft ein Verfahren zur Anzeige von patientenbezogenen Diagnosen chronischer Krankheiten, ein Datenverarbeitungssystem und ein Computerprogrammprodukt.

Medizinische Informationssysteme dokumentieren unter anderem vielfältige, patientenbezogene, administrative und medizinische Daten. Obwohl jedoch durch die Verwendung von medizinischen Informationssystemen die einem behandeltem Arzt zur Verfügung stehende Möglichkeiten zur Dokumentation von Patientendaten eine im Wesentlichen lückenlose Aufzeichnung und Speicherung der Patientendaten ermöglichen, so ergibt sich aufgrund oftmals vorhandener Zeitprobleme in Arztpraxen und Krankenhäusern das Problem, dass ein behandelnder Arzt nur selten dazu in der Lage sein wird, durch Durchsicht der Patientenakte eines Patienten vor Beginn eines Behandlungstermins dieses Patienten sich einen vollständigen Überblick über den Behandlungsablauf des Patienten zu machen. Ein behandelnder Arzt hat in diesem Fall oftmals lediglich dafür Zeit, sich intensiv mit Gesundheitsstörungen und Diagnosen des Patienten zu beschäftigen, welche lediglich in der jüngsten Vergangenheit aufgetreten waren.

Eine weitere Ursache für die beschränkte Rückschaufähigkeit des Arztes findet sich jedoch auch in dem typischen Aufbau von grafischen Benutzeroberflächen medizinischer Informationssysteme. Zur Illustration eines Patientendatenblatts zeigen solche Informationssysteme aufgrund der begrenzten Darstellungsmöglichkeiten einer grafischen Benutzeroberfläche lediglich die zuletzt eingegebenen medizinischen Diagnosen und Hinweise an. Zwar könnte ein Arzt durch "Blättern" in der Patientenhistorie, das heißt durch Bewegung einer Scrollbar Zugriff auf weitere, in einem längeren Zeitraum zurückliegende Diagnosen erlangen, was er jedoch wie eingangs geschildert, aus Zeitgründen nur selten detailliert tun kann. Der Arzt kann sich somit unter Verwendung medizinischer Informationssysteme in kurzer Zeit keinen allgemeinen Überblick über die Krankheitshistorie eines Patienten machen.

Als Konsequenz daraus ergibt sich die Problematik, dass insbesondere chronische Krankheiten, welche sich durch z.B. wiederkehrende Krankheitssymptome äußern, gegebenenfalls nicht durch den behandelnden Arzt erkannt werden, da der Arzt aufgrund der Darstellung der Behandlungshistorie des Patienten auf seiner grafischen Benutzeroberfläche nicht in einfacher Weise Informationen darüber erlangt, ob sich z.B. ständig wiederkehrende Krankheitssymptome und Diagnosen in der Krankheitshistorie des Patienten befinden, welche einen Hinweis auf das Vorhandensein einer entsprechenden chronischen Erkrankung geben könnten. Eine Überprüfung der Behandlungshistorie hinsichtlich der Symptome und Befunde des Patienten müsste damit in zeitlich aufwändiger Weise individuell für jeden Patienten vor einem Behandlungstermin erfolgen.

Damit liegt die Erfindung die technische Aufgabe zugrunde, einen Benutzer eines medizinischen Informationssystems in die Lage zu versetzen, eine Analyse von Patientendaten hinsichtlich des Vorhandenseins chronischer Krankheiten in effizienterer und schnellerer Weise durchzuführen.

Die der Erfindung zugrunde liegende Aufgabe wird mit den Merkmalen der unabhängigen Patentansprüche gelöst. Bevorzugte Ausführungsformen der Erfindung sind in den abhängigen Patentansprüchen angegeben.

Erfindungsgemäß wird ein Verfahren zur Anzeige von patientenbezogenen Diagnosen chronischer Krankheiten auf einer grafischer Benutzeroberfläche eines Datenverarbeitungssystems geschaffen, wobei die grafische Benutzeroberfläche zumindest ein erstes und ein zweites Anzeigefenster aufweist. Das Verfahren umfasst dabei zunächst den Schritt des Anzeigens von zumindest einem Teil von Patientendaten eines Patienten in dem ersten Anzeigefenster, wobei die angezeigten Patientendaten in dem ersten Anzeigefenster zeilenweise angezeigt werden, wobei das erste Anzeigefenster zur zeilenweise Nachführung der anzuzeigenden Patientendaten durch eine Scrollbar ausgebildet ist. Danach erfolgt der Zugriff auf eine erste Datenbank, wobei diese erste Datenbank medizinische Diagnoseobjekte enthält, wobei die medizinischen Diagnoseobjekte mit ersten Abfragebedingungen bezüglich der Patientendaten des Patienten verknüpft sind. Daraufhin erfolgt die Überprüfung, ob zumindest eine der ersten Abfragebedingungen für die Patientendaten erfüllt ist. Ist dies der Fall, erfolgt das Anzeigen eines Anzeigeelements auf der grafischen Benutzeroberfläche, wobei das Anzeigeelement zumindest eines der Diagnoseobjekte aufweist, für welche die erste Abfragebedingung erfüllt ist. Daraufhin erfolgt die Ausgabe einer Benutzerabfrage auf der grafischen Benutzeroberfläche, ob eine mit dem Diagnoseobjekt verknüpfte medizinische Diagnose als chronische Dauerdiagnose übernommen werden soll. Wenn die mit dem Diagnoseobjekt verknüpfte medizinische Diagnose als Dauerdiagnose übernommen werden soll, so erfolgt eine dauerhaft sichtbare Anzeige der Dauerdiagnose in dem zweiten Anzeigefenster unabhängig von der Position des Scrollbars.

Unter Patientendaten werden im Folgenden jegliche Art von Informationen verstanden, welche hinsichtlich eines Patienten erfasst wurden. Dazu gehören nebst strukturierten und freitextlichen Textdaten auch elektronische Bilddaten sowie medizinische Messdaten jeglicher Art. Unter strukturierten Patientendaten werden Patientendaten verstanden, welche gemäß einer zuvor festgelegten Norm bzw. Klassifikation bereitgestellt wurden. Darunter fällt insbesondere die Verwendung der internationalen statistischen Klassifikation der Krankheiten und verwandter Gesundheitsprobleme (ICD), wie auch Pharmazentralnummern (PZN) oder die spezifischen Inhalte von Krankenverordnungs-(KV)formularen, wie Überweisungen, Einweisungen, Arbeitsunfähigkeitsbescheinigungen oder Ähnlichem.

Das erfindungsgemäße Verfahren hat den Vorteil, dass ein behandelnder Arzt darin unterstützt wird, in schneller und effizienter Weise Diagnosen hinsichtlich chronischer Krankheiten eines Patienten zu stellen. Der behandelnde Arzt muss nicht mehr in aufwändiger Weise eine Durchsicht aller ihm zur Verfügung stehender Patientendaten eines Patienten vornehmen, zumal dies, wie bereits oben angemerkt, üblicherweise aus Zeitgründen nicht möglich ist.

Detektiert nach dem obig genannten Verfahren das Datenverarbeitungssystem in der persönlichen Patientenakte eines Patienten eine Erfüllung von mindestens einer der ersten Abfragebedingungen, schlägt das Datenverarbeitungssystem dem behandelnden Arzt vor, die mit dieser Abfragebedingung verknüpfte medizinische Diagnose einer chronischen Krankheit in dauerhafter Weise in die Rubrik "Dauerdiagnosen" zu übernehmen. Die Dauerdiagnosen werden dabei in einem von dem ersten Anzeigefenster separaten zweiten Anzeigefenster dauerhaft sichtbar und unabhängig von der Position der Scrollbar angezeigt. Damit ist ein behandelnder Arzt selbst bei Darstellung von lediglich dem letzen Eintrag in die Patientenakte im ersten Anzeigefenster dazu in der Lage, auch bei einem erneuten Aufrufen der Patientenakte sofort über das Vorhandensein einer solch entscheidenden Diagnose einer chronischen Krankheit informiert zu sein. Es sei angemerkt, dass zu diesem Zweck vorzugsweise das Verfahren ferner das Speichern der Dauerdiagnose in der zweiten Datenbank, in welcher auch die Patientendaten enthalten sind, mit den Patientendaten zusammen verknüpft umfasst.

Es sei noch darauf hingewiesen, dass unter "Diagnoseobjekt" jegliche Art von Information verstanden wird, welche es ermöglicht, eine medizinische Diagnose zu beschreiben. Hierzu zählen Freitextinformationen, welche beispielsweise die Diagnose namentlich benennen, oder welche detailliert ein mit der chronischen Erkrankung einhergehendes Krankheitsbild beschreiben. Ferner gehören zu Diagnoseobjekten jedoch auch die bereits obig erwähnten ICD-Codes oder allgemein strukturierte Informationen.

Nach einer Ausführungsform der Erfindung weist die grafische Benutzeroberfläche ferner ein drittes Anzeigefenster auf, wobei das Verfahren, wenn die mit dem Diagnoseobjekt verknüpfte medizinische Diagnose als Dauerdiagnose übernommen werden soll, ferner den Schritt umfasst des Zugriffs auf eine dritte Datenbank, wobei die dritte Datenbank medizinische Medikamentenobjekte enthält, wobei die medizinischen Medikamentenobjekte mit zweiten Abfragebedingungen bezüglich der empfangenen Patientendaten verknüpft sind. Daraufhin erfolgte eine Überprüfung, ob zumindest eine der zweiten Abfragebedingungen für die Patientendaten erfüllt ist. Ist dies der Fall, wird ein weiteres Anzeigeelement auf der grafischen Benutzeroberfläche angezeigt, wobei dieses weitere Anzeigeelement zumindest eines der Medikamentenobjekte aufweist, für welches die Abfragebedingung erfüllt ist. Daraufhin erfolgt die Ausgabe einer weiteren Benutzerabfrage auf der grafischen Benutzeroberfläche, ob ein mit dem Medikamentenobjekt verknüpftes Medikament als Präparat zur Dauermedikation übernommen werden soll. Soll das mit dem Medikamentenobjekt verknüpftes Medikament als Präparat zur Dauermedikation übernommen werden, so erfolgt nach einer entsprechenden Benutzerbestätigung eine dauerhaft sichtbare Anzeige der Dauermedikation in dem dritten Anzeigefenster, ebenfalls unabhängig von der Position der Scrollbar.

In anderen Worten folgt, wenn die mit dem Diagnoseobjekt verknüpfte medizinische Diagnose als Dauerdiagnose übernommen werden soll, das heißt wenn eine chronische Erkrankung durch den Arzt als gesichert gewertet wird, der weitere Schritt einer Überprüfung, ob bereits zur Behandlung der chronischen Krankheit verwendete Medikamente gemäß der Patientenakte, das heißt der Patientendaten, dem Patienten zuvor verordnet wurden. Detektiert das System eine relevante chronische Erkrankung und finden sich Wirkstoffe bzw. Medikamente in der individuellen Patientenakte, die zu diesen chronischen Erkrankungen passen, so wird dem Arzt die Übernahme des jeweiligen Präparats in die Rubrik "Dauermedikation" im dritten Anzeigefenster vorgeschlagen. Auch hier entfällt wieder eine aufwändige und zeitintensive Suche nach entsprechenden Medikamenten oder Wirkstoffen in den Patientendaten, womit der Arzt wiederum in die Lage versetzt wird, in schnellerer und effizienter Weise eine Analyse der Patientendaten, welche in einer entsprechenden Datenbank gespeichert sind, durchzuführen.

Nach einer weiteren Ausführungsform der Erfindung handelt es sich bei der ersten und/oder dritten Datenbank um eine dem Datenverarbeitungssystem externe Datenbank, es ist jedoch auch möglich, das die erste und/oder dritte Datenbank in dem Datenverarbeitungssystem selbst enthalten ist. Nach einer bevorzugten Ausführungsform jedoch befinden sich die Patientendaten auf einer zweiten Datenbank, beispielsweise einem Arztinformationssystem. Die erste Datenbank ist mit der dritten Datenbank identisch und wird beispielsweise zusammen mit dem obig genannten Datenverarbeitungssystem zur Verfügung gestellt. In einer weiteren alternativen Variante ist es auch möglich, dass die zweite Datenbank in einem Arztinformationssystem enthalten ist, wobei dieses Arztinformationssystem das erfindungsgemäße Verfahren wie obig beschrieben durchführen kann. Zur Durchführung des Verfahrens greift das Arztinformationssystem über ein Netzwerk auf einen Webservice zu, welcher von einem Server abgerufen werden kann. Dieser Webservice stellt beispielsweise in Form eines Servlets einen Dienst zur Verfügung, welcher es ermöglicht, das erfindungsgemäße Verfahren auf die Patientendaten anzuwenden. Allgemein ist es möglich, dass der Webservice zwar auf die erste und dritte Datenbank zugreift, welche externe Datenbanken hinsichtlich des Arztinformationssystems darstellen, wobei jedoch der Webservice entweder auf dem Arztinformationssystem ausgeführt werden kann, oder serverseitig auf einem Server, welcher von einem medizinischen Dienstleister betrieben wird. Die erste und dritte Datenbank kann dabei diesem Server des medizinischen Dienstleisters zugeordnet sein. Unabhängig von der Verwendung von Webservices kann das erfindungsgemäße Verfahren auch auf einem externen Server durchgeführt werden, wobei die graphische Benutzeroberfläche Teil eines Clients ist, über welchen Patientendaten eingegeben werden und welcher von einem entsprechenden Arzt bedienbar ist.

Nach einer weiteren Ausführungsform der Erfindung sind die Abfragebedingungen mit einer Zeitkonstante für ein maximales Alter verknüpft, wobei die Überprüfung, ob zumindest eine der ersten und/oder zweiten Abfragebedingungen erfüllt ist, nur auf die Patientendaten angewendet wird, welche einen jüngeren Zeitstempel als das maximale Alter aufweisen. Damit wird zuverlässig vermieden, dass das Auftreten gleichartiger Diagnosen, welche jedoch im großen zeitlichen Abstand zueinander erfolgt sind, fälschlicherweise als Diagnose für eine chronische Erkrankung aufgrund ihres wiederkehrenden Auftretens fälschlicherweise interpretiert wird. Selbiges gilt auch hinsichtlich einer Medikamentierung, da hier nur solche Medikamente eine Rolle spielen, welche innerhalb eines vorbestimmten Zeitraums in wiederkehrender Weise dem Patienten verordnet wurden. Dieser vorbestimmte Zeitraum ist zunächst systemseitig vorgegeben, kann jedoch auch vom Arzt in entsprechender Weise angepasst werden. Außerdem ist dieser vordefinierte Zeitraum vorzugsweise abhängig von der Art der medizinischen Diagnose, sodass die Zeitkonstante für jede Abfragebedingung individuell festgelegt ist. Nichtsdestotrotz ist es möglich, eine globale Höchstgrenze hinsichtlich des Alters der in Betracht kommenden Patientendaten festzulegen.

Nach einer weiteren Ausführungsform der Erfindung umfasst das Verfahren ferner den Schritt der Aufbereitung der Patientendaten, wobei die Überprüfung, ob zumindest eine der ersten und/oder zweiten Abfragebedingungen erfüllt ist, nur für die aufbereiteten Patientendaten durchgeführt wird, wobei die Datenaufbereitung die Filterung von strukturierten Daten aus den Patientendaten umfasst. Zusätzlich ist es nach einer Ausführungsform der Erfindung auch möglich, dass die Datenaufbereitung ferner die semantische Umwandlung von Freitextinformationen der Patientendaten in strukturierte Daten umfasst. Dies hat den Vorteil, dass aus einer großen Datenmenge zunächst in effizienter Weise diejenigen Daten gefiltert werden können, über welche eine entsprechende Analyse hinsichtlich des Vorhandenseins einer chronischen Erkrankung überhaupt durchgeführt werden kann. Durch die semantische Interpretation von Freitextinformationen wird zudem sichergestellt, dass eine vom Arzt lediglich in allgemeinen Worten umschriebene medizinische Diagnose maschineninterpretierbar beispielsweise in einen ICD-Code umgewandelt wird, sodass daraufhin die Abfragebedingungen entsprechend angewendet werden können. Dies hat den weiteren Vorteil, dass die Abfragebedingungen einfach gehalten werden können, da somit beispielsweise eine zusätzliche Abfrage auf freitextliche Begriffe wie "Kopfschmerzen" oder "Schnupfen" nicht nötig ist. Dies reduziert zum einen den Programmieraufwand zur Erstellung entsprechender Abfragen, wobei die Redundanz entsprechender Abfragen, beispielsweise einmal als Freitextinformation und einmal als ICD-Code, wesentlich reduziert wird. Damit wird eine entsprechende Abfrage wesentlich beschleunigt. Insbesondere bei der Verwendung des erfindungsgemäßen Verfahrens durch einen Server-Client-System stellt dies einen wesentlichen Vorteil dar, da für entsprechende Abfragen die erste und dritte Datenbank in ihrem Umfang relativ klein gehalten werden können - die zu übertragende Datenmenge bzw. die Anzahl an durchzuführenden Abfragen wird damit drastisch reduziert.

Nach einer Ausführungsform der Erfindung werden die aufbereiteten Patientendaten in einer vierten Datenbank gespeichert, wobei die Überprüfung, ob zumindest einer der ersten und/oder zweiten Abfragebedingungen für die Patientendaten erfüllt ist, durch Zugriff auf die vierte Datenbank erfolgt. Bei der vierten Datenbank kann es sich wiederum um eine dem Datenverarbeitungssystem externe Datenbank handeln. Jedoch ist vorzugsweise die vierte Datenbank ein Cache-Speicher des Datenverarbeitungssystems, sodass in sehr schneller Weise eine Abfrage auf die relevanten Patientendaten durchgeführt werden kann.

Nach einer weiteren Ausführungsform der Erfindung erfolgt die Überprüfung, ob zumindest eine der ersten und/oder zweiten Abfragebedingungen für die Patientendaten erfüllt ist, automatisch nach dem Anzeigen des zumindest einen Teils des Patientendaten im ersten Anzeigefenster. Außerdem wird das Verfahren vorzugsweise in Echtzeit durchgeführt, wobei das Verfahren ferner den Schritt umfasst des Empfangens von neuen Patientendaten durch eine entsprechende Benutzereingabe. Zusammengefasst bietet sich dadurch den Vorteil, dass ein behandelnder Arzt grundsätzlich unmittelbar und direkt entweder nach Öffnen der Patientenakte oder nach Eingabe von entsprechenden Patientendaten in die Patientenakte zuverlässig darüber informiert wird, ob sein Patient dahingehend gefährdet ist, eine chronische Erkrankung aufzuweisen.

Nach einer weiteren Ausführungsform der Erfindung handelt es sich bei dem Anzeigeelement um ein Popup.

Nach einer weiteren Ausführungsform der Erfindung handelt es sich bei den ersten Abfragebedingungen um Abfragen für das Vorhandensein von einer oder mehrerer in den Patientendaten enthaltenen Diagnosen, wobei die medizinischen Diagnoseobjekte mit Wahrscheinlichkeiten für das Vorhandensein einer chronischen Erkrankung verknüpft sind, wobei die Diagnoseobjekte ferner mit einem Schwellwert verknüpft sind, wobei eine erste Abfragebedingung für ein Diagnoseobjekt nur dann erfüllt ist, wenn die Gesamtwahrscheinlichkeit für das Vorhandensein einer chronischen Erkrankung höher ist als der mit dem Diagnoseobjekt verknüpfte Schwellwert. Somit ist es auch möglich, verschiedene Krankheitssymptome in ihrer Gesamtheit dahingehend zu analysieren, ob diese einen Hinweis auf das Vorhandensein einer chronischen Erkrankung geben. Auch hier zeigt sich wieder der Vorteil des erfindungsgemäßen Verfahrens, nämlich in schneller und effizienter Weise eine Analyse der Patientendaten durchzuführen. Durch das erfindungsgemäße Verfahren werden somit auch komplexe Zusammenhänge hinsichtlich von Gesundheitssymptomen erfasst, welche ein behandelnder Arzt im Falle einer umfangreichen Patientenakte in ihrer Gesamtheit nicht erfassen könnte.

Nach einer weiteren Ausführungsform der Erfindung wird die Benutzerabfrage, ob eine mit dem Diagnoseobjekt verknüpfte medizinische Diagnose als chronische Dauerdiagnose übernommen werden soll, in dem Anzeigeelement ausgegeben. Außerdem wird nach einer Ausführungsform der Erfindung die Überprüfung, ob zumindest eine der ersten Abfragebedingungen für die Patientendaten erfüllt ist, in der Reihenfolge abnehmender Trefferwahrscheinlichkeiten der Abfragebedingungen durchgeführt. Somit müssen nicht sämtliche Abfragen, welche zur Verfügung stehen, auf die Patientendaten angewendet werden, sondern diese Abfragen können hierarchisch gegliedert werden. Eine solche Hierarchie kann beispielsweise angeben, dass entsprechende Abfragen hinsichtlich des Vorhandenseins von Diagnosen für chronische Erkrankungen nur dann durchgeführt werden, wenn der entsprechende Patient in eine bestimmte Altersgruppe fällt.

Nach einer weiteren Ausführungsform der Erfindung umfasst das Verfahren ferner den Schritt des Zugriffs auf die erste Datenbank, wobei die erste Datenbank ferner Regeln enthält, wobei die Regeln zur Berechnung von Grundrisiken für medizinische Diagnosen ausgebildet sind, wobei in der ersten Datenbank ferner die medizinischen Diagnosen mit medizinischen Symptomen verknüpft gespeichert sind. Daraufhin erfolgt das Anwenden der Regeln auf die Patientendaten und Berechnen zumindest eines ersten Grundrisikos für eine erste medizinische Diagnose, wenn zumindest eine der Regeln auf die Patientendaten anwendbar ist. Daraufhin erfolgt die Ausgabe des ersten Grundrisikos für die erste medizinische Diagnose zusammen mit der ersten medizinischen Diagnose auf der grafischen Benutzeroberfläche und die Ausgabe der Benutzerabfrage, ob für diese erste medizinische Diagnose eine interaktive Patientendatenanalyse durchgeführt werden soll. Im Falle dessen, dass für die erste medizinische Diagnose eine interaktive Patientendatenanalyse durchgeführt werden soll, in anderen Worten ein Benutzer eine entsprechende Eingabe am Datenverarbeitungssystem macht, so erfolgt eine Ausgabe einer Symptom-Benutzerabfrage, welche mit der ersten medizinischen Diagnose verknüpften medizinischer Symptome für eine weitere Analyse der Patientendaten verwendet werden soll. Daraufhin erfolgt das Anwenden der Regeln auf die Patientendaten und die vom Benutzer in der Symptom-Benutzerabfrage gewählten medizinischen Symptome und ein Berechnen zumindest eines zweiten Grundrisikos für eine zweite medizinische Diagnose. Schließlich erfolgt die Ausgabe des zweiten Grundrisikos zusammen mit der zweiten medizinischen Diagnose auf der grafischen Benutzeroberfläche.

Dies hat den Vorteil, dass ein behandelnder Arzt in die Lage versetzt wird, verschiedene - nicht zwingend chronische - medizinische Diagnosen in Ihrer Gesamtheit zusammenhängend zu berücksichtigen. Damit kann er in anderen Worten eine Analyse der Patientendaten in schnellerer und effizienterer Weise durchführen. Außerdem ermöglicht das Verfahren, dass ein Arzt automatisch auf mögliche medizinische Diagnosen hingewiesen wird, welche bei manueller Durchsicht der Patientendaten nicht zu erkennen sind, da hierfür komplexe Zusammenhänge zwischen medizinischen Befunden berücksichtigt werden müssen. Durch das genannte Verfahren werden somit einem Arzt verschiedene Grundrisiken angezeigt, die für das Vorhandensein verschiedener medizinischer Diagnosen gegeben sind. Ist der Arzt der Auffassung, dass eine mögliche Diagnose eine hohe Relevanz im vorliegenden Behandlungsfall haben könnte, so kann er durch Bestätigung der Benutzerabfrage, ob für die erste medizinische Diagnose eine interaktive Patientendatenanalyse durchgeführt werden soll, in geführter Weise rasch und effektiv eine Bestimmung dahingehend durchführen, ob eine angezeigte medizinische Diagnose tatsächlich relevant ist oder nicht. In anderen Worten kann damit ein Verdacht für eine bestimmte Diagnose erhärtet oder verworfen werden. Somit wird insgesamt gewährleistet, dass die Interaktionszeit des Arztes mit dem Datenverarbeitungssystem wesentlich verkürzt wird. Selbiges gilt auch für die Zugriffsdauer auf eine Datenbank, auf welcher die entsprechenden Patientendaten abgelegt sind.

Nachdem ein Benutzer in der Symptombenutzerabfrage verschiedene medizinische Symptome, welche mit der ersten medizinischen Diagnose verknüpft sind, zur weiteren Analyse der Patientendaten ausgewählt hat, erfolgt ein erneutes Anwenden der Regeln auf die Patientendaten und die vom Benutzer gewählten medizinischen Symptome, um damit ein Grundrisiko unter Verwendung dieser neuer Kriterien berichtigt zu berechnen.

Nach einer Ausführungsform der Erfindung werden medizinische Diagnosen erst ab einem vorbestimmten Grundrisiko ausgegeben. Außerdem werden vorzugsweise medizinische Diagnosen sortiert nach dem Grundrisiko ausgegeben. Damit ist gewährleistet, dass ein Benutzer des Datenverarbeitungssystems, d.h. ein behandelnder Arzt, nicht in unnötiger Weise mit irrelevanten medizinischen Diagnosen konfrontiert wird.

Nach einer Ausführungsform der Erfindung wird das erste und zweite Grundrisiko in Form einer Tachoscheibe angezeigt. Vorzugsweise wird dabei das Grundrisiko mit farbigen Abstufungen auf der Skala der Tachoscheibe angezeigt. Zusätzlich wird nach einer Ausführungsform der Erfindung das Grundrisiko als Risikowahrscheinlichkeit in Form eines Zahlenwerts zusammen mit der Tachoscheibe angezeigt. Damit ist ein Benutzer in der Lage, in intuitiver Weise das Risiko für das Vorliegen einer bestimmten medizinischen Diagnose zu erfassen, um daraufhin in zeiteffizienter Weise eine entsprechende Entscheidung darüber zu treffen, ob diese Diagnose im Detail weiterverfolgt werden soll oder nicht.

Nach einer weiteren Ausführungsform der Erfindung wird zusammen mit dem ersten Grundrisiko ein erstes Bedienelement bereitgestellt, wobei das erste Bedienelement für eine Benutzerbestätigung ausgebildet ist, wobei im Falle einer Benutzerbestätigung über das erste Bedienelement die erste medizinische Diagnose und/oder die medizinischen Symptome mit den Patientendaten verknüpft in der zweiten Datenbank gespeichert wird. Damit wird ein Arzt in die Lage versetzt, eine ihm als gesichert erscheinende medizinische Diagnose, gegebenenfalls zusammen mit den von ihm eingegebenen Symptomen, mit in die Patientendatenbank aufzunehmen, sodass bei einem erneuten Aufrufen der Patientenakte ein solch medizinischer Befund als Teil der Patientenakte dem Arzt erneut zugänglich ist.

Nach einer weiteren Ausführungsform der Erfindung wird zusammen mit dem zweiten Grundrisiko ein zweites Bedienelement bereitgestellt, wobei das zweite Bedienelement für eine Benutzerbestätigung ausgebildet ist, wobei im Fall einer Benutzerbestätigung über das zweite Bedienelement das zweite Grundrisiko und die zweite medizinische Diagnose als neues erstes Grundrisiko und als neue erste medizinische Diagnose auf der grafischen Benutzeroberfläche ausgegeben wird. In anderen Worten wird hiermit die Möglichkeit gegeben, die durch die zusätzliche Eingabe von Symptomen präzisierte Diagnose in jener Übersicht zu aktualisieren, welche ursprünglich mit der Ausgabe des ersten Grundrisikos für die erste medizinische Diagnose zusammen mit der ersten medizinischen Diagnose erzeugt wurde. Dies ist insbesondere für den Fall relevant, dass nicht nur eine einzelne medizinische Diagnose mit einem entsprechenden Grundrisiko versehen ursprünglich angezeigt wurde, sondern ein Satz von verschiedenen Diagnosen. Durch die Durchführung der Symptombenutzerabfrage wird eine der Diagnosen präzisiert, wodurch sich jedoch auch die weiteren in dieser Übersicht vorhandenen Diagnosen aufgrund der zusätzlichen Angabe der Symptome verändert haben können. Durch eine dynamische Anpassung der ersten Grundrisiken auf Basis der Patientendaten und sämtlicher eingegebener Symptome wird so in übersichtlicher Weise eine hochpräzise und aktualisierte Übersicht über mögliche Risikowahrscheinlichkeiten für Symptome angezeigt.

Nach einer Ausführungsform der Erfindung werden nach jeder Benutzerauswahl eines weiteren medizinischen Symptoms erneut die Regeln auf die Patientendaten und die bisher vom Benutzer gewählten medizinischen Symptome angewendet. Daraufhin erfolgt ein erneutes Berechnen von zumindest einem neuen zweiten Grundrisiko für eine neue zweite medizinische Diagnose in dynamischer Weise, gefolgt von einer aktualisierten Ausgabe des erneut berechneten neuen zweiten Grundrisikos zusammen mit der neuen zweiten medizinischen Diagnose auf der grafischen Benutzeroberfläche. Schließlich erfolgt eine aktualisierte Ausgabe der Benutzerabfrage, welche mit der neuen zweiten medizinischen Diagnose verknüpften medizinischen Symptome für eine weitere Analyse der Patientendaten verwendet werden sollen. Damit ist der Arzt in der Lage, sofort zu erkennen, welche Signifikanz die spezielle Angabe eines einzelnen Symptoms auf mögliche Diagnosen hinsichtlich deren Grundrisiko hat.

Nach einer weiteren Ausführungsform der Erfindung erfolgt mit der aktualisierten Ausgabe des erneut berechneten neuen zweiten Grundrisikos eine erneute aktualisierte Ausgabe der Symptombenutzerabfrage, wobei die aktualisierte Ausgabe der Symptombenutzerabfrage angibt, welche der mit der neuen zweiten medizinischen Diagnose verknüpften medizinischen Symptome für eine weitere Analyse der Patientendaten verwendet werden sollen, wobei zuvor vom Benutzer gewählte medizinische Symptome in der aktualisierten Ausgabe der Symptombenutzerabfrage beibehalten werden. In anderen Worten wird dadurch die Liste der auswählbaren möglichen medizinischen Symptome weiter eingeschränkt oder durch weitere mögliche auswählbare Symptome dynamisch ergänzt. Dies ist beispielsweise dann relevant, wenn die kombinierte Auswertung von Patientendaten und gewählter Symptome einen Hinweis darauf geben, dass eine mögliche Erkrankung vorliegt, welche jedoch lediglich bei Berücksichtigung weiterer, bisher nicht angezeigter Symptome, für eine Grundrisikoberechnung berücksichtigt werden kann.

Nach einer weiteren Ausführungsform der Erfindung erfolgt die Symptombenutzerabfrage in Form einer Checkboxliste.

Nach einer Ausführungsform der Erfindung wird zumindest ein Teil der Patientendaten in dem ersten Anzeigefenster der grafischen Benutzeroberfläche bereitgestellt, wobei das erste und zweite Grundrisiko auf eine erste und zweite medizinische Diagnose zusammen mit der medizinischen Diagnose auf der grafischen Benutzeroberfläche in dem Popup ausgegeben wird.

Nach einer weiteren Ausführungsform der Erfindung erfolgt das Anwenden der Regeln auf die Patientendaten automatisch nach dem Anzeigen der Patientendaten im ersten Anzeigefenster.

In einem weiteren Aspekt betrifft die Erfindung ein Datenverarbeitungssystem mit einer grafischen Benutzeroberfläche, wobei das Datenverarbeitungssystem zur Durchführung des Verfahrens zur Anzeige von patientenbezogenen Diagnosen chronischer Krankheiten ausgebildet ist.

In einem weiteren Aspekt betrifft die Erfindung ein Computerprogrammprodukt mit von einem Prozessor ausführbaren Instruktionen zur Durchführung des erfindungsgemäßen Verfahrens zur Anzeige von patientenbezogenen Diagnosen chronischer Krankheiten.

Im Folgenden werden Ausführungsformen der Erfindung anhand der Zeichnungen näher erläutert. Es zeigen:
- Figur 1: ein Blockdiagramm eines erfindungsgemäßen Datenverarbeitungssystems,
- Figur 2: eine schematische Ansicht einer grafischen Benutzeroberfläche,
- Figur 3: ein Flussdiagramm eines Verfahrens zur Anzeige von patientenbezogenen Diagnosen chronischer Krankheiten,
- Figur 4: ein Verfahren zur medizinischer Diagnoseunterstützung für Patientendaten eines Patienten,
- Figur 5: Schritte eines Verfahrens zur medizinischen Diagnoseunterstützung für Patientendaten eines Patienten.

Im Folgenden sind einander ähnliche Elemente mit gleichen Bezugszeichen gekennzeichnet.

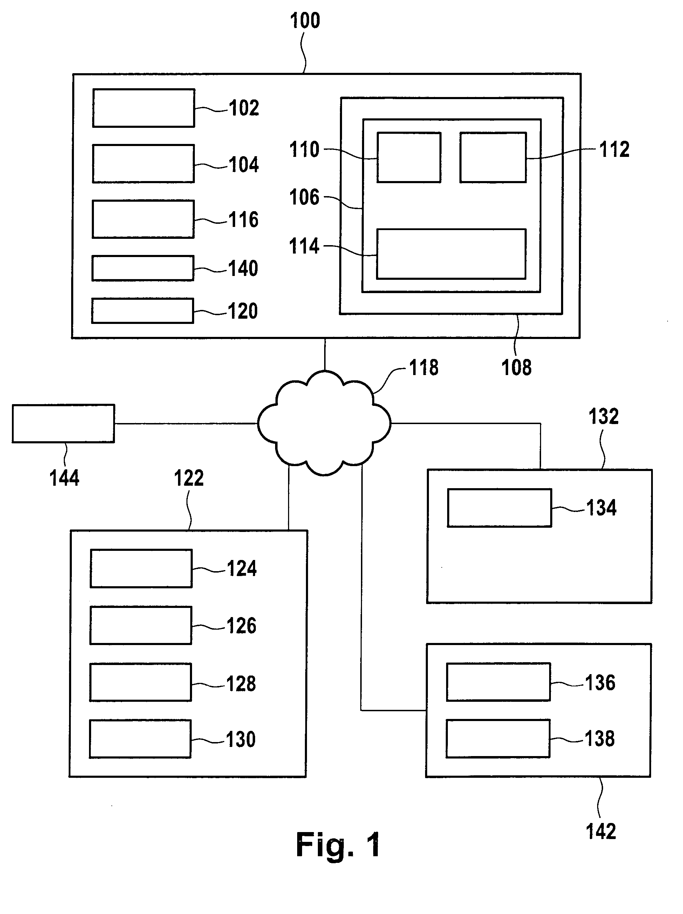

Die Figur 1 zeigt ein Blockdiagramm eines erfindungsgemäßen Datenverarbeitungssystems 100. Das Datenverarbeitungssystem 100 weist einen Prozessor 104 und Eingabemittel 102 auf, wie z.B. eine Maus, Tastatur usw. Als Eingabemittel können auch medizintechnische Geräte dienen, mittels welcher entsprechende medizinische Bild und/oder Messdaten eines Patienten erfasst und gespeichert werden können. Ferner weist das Datenverarbeitungssystem 100 einen Speicher 116 auf, in welchem sich ein computerausführbarer Code für ein Anwendungsprogramm, beispielsweise zur Durchführung des erfindungsgemäßen Verfahrens, befindet. Des Weiteren weist das Datenverarbeitungssystem 100 eine grafische Benutzeroberfläche 106 auf, welche auf einer entsprechenden Anzeigevorrichtung 108 ausgegeben wird. Bei dieser Anzeigevorrichtung 108 kann es sich beispielsweise um einen LCD- oder CRT-Bildschirm handeln.

Über eine Schnittstelle 120 kann das Datenverarbeitungssystem 100 mit Datenbanken 122, 132 und 142, beispielsweise über das Netzwerk 118, kommunizieren. Die Datenbanken 122, 132 und 142 können jedoch auch Teil des Datenverarbeitungssystems 100 selbst sein. Außerdem kann der Code zur Ausführung durch den Prozessor 104 auch von einem Server 144 abgerufen werden, wobei in diesem Fall der Code zur Durchführung des erfindungsgemäßen Verfahrens z.B. mittels eines Webservices zur Verfügung gestellt wird. Der Code kann entweder auf den Server 144 oder aber im Datenverarbeitungssystem 100 ausgeführt werden.

Zur Durchführung des Verfahrens zur Anzeige von patientenbezogenen Diagnosen chronischer Krankheiten auf der grafischen Benutzeroberfläche 106 sei im Folgenden davon ausgegangen, dass es sich bei den Datenbanken 122, 132 und 142 um externe Datenbanken handelt und dass ferner das Verfahren direkt auf dem Datenverarbeitungssystem 100 durch den Prozessor 104 durchgeführt wird. Hierzu öffnet ein behandelnder Arzt zunächst eine Patientenakte. Diese Patientenakte enthält Patientendaten 134, welche in der Datenbank 132 gespeichert sind. Zu diesem Zweck werden nun zunächst die Patientendaten 134 über das Netzwerk 118 an das Datenverarbeitungssystem 100 übermittelt. Die zuletzt eingegebenen Patientendaten werden daraufhin in dem Anzeigefenster 114 zeilenweise dargestellt, wobei dieses Anzeigefenster eine Scrollbar aufweist. Damit ist der Arzt unter Bewegung der Scrollbar in der Lage, sämtliche Einträge der Patientendaten durchzublättern.

Da jedoch typischerweise ein behandelnder Arzt aus Zeitgründen nicht dazu in der Lage ist, sich in zuverlässiger Weise einen Überblick über die gesamte Krankheitshistorie eines Patienten zu verschaffen, wird nun durch das Datenverarbeitungssystem 100 bzw. dessen Prozessor 104 nach Öffnen der Patientenakte zunächst so vorgegangen, dass über das Netzwerk 118 auf die Datenbank 122 zugegriffen wird. Diese Datenbank 122 enthält medizinische Diagnoseobjekte 124 und Abfragebedingungen 126. Durch Überprüfung, ob zumindest einer der Abfragebedingungen 126 für die Patientendaten 134 erfüllt ist, kann das Datenverarbeitungssystem 100 ermitteln, ob gegebenenfalls eine hohe Wahrscheinlichkeit für das Vorhandensein einer chronischen Krankheit des Patienten gegeben ist. Ist eine der Abfragebedingungen 126 für die Patientendaten 134 erfüllt, so erfolgt das Anzeigen eines Anzeigeelements, beispielsweise eines Popups, auf der grafischen Benutzeroberfläche 106. Dieses Popup enthält weiterführende Informationen hinsichtlich der Möglichkeit des Vorhandenseins einer chronischen Erkrankung, und somit insbesondere Informationen, welche in dem medizinischen Diagnoseobjekt 124 enthalten sind. Dies kann beispielsweise ein ICD-Code oder der Name einer entsprechenden chronischen Erkrankung sein. Außerdem können zusätzlich weiterführende Informationen und gegebenenfalls auch Verknüpfungen in Form von Hyperlinks zu weiteren Datenbanken angegeben werden, mittels derer sich der behandelnde Arzt in detaillierte Weise weiter über das entsprechende chronische Krankheitsbild informieren kann.

Nachdem ein entsprechendes Anzeigeelement in Form beispielsweise eines Popups oder auch in Form eines beliebigen anderen Anzeigeelements auf der grafischen Benutzeroberfläche angezeigt wurde, bietet das Datenverarbeitungssystem 100 dem behandelnden Arzt die Möglichkeit, die entsprechende chronische Krankheit in die Rubrik "Dauerdiagnose" zu nehmen, das heißt diese Diagnose in permanenter Weise im Anzeigefenster 110 der grafischen Benutzeroberfläche 106 anzeigen zu lassen, und zwar unabhängig von einer Scroll-Bewegung innerhalb der verschiedenen Zeilen der Patientendaten im Anzeigefenster 114. Wird Entsprechendes vom Arzt bestätigt, so erfolgt daraufhin vorzugsweise diese permanente Anzeige der medizinischen Diagnose, beispielsweise in Form eines ICD-Codes, im Anzeigefenster 110 und außerdem eine Speicherung dieser Anzeigeoption für den Patienten in dessen Patientenakte in der Datenbank 132. In anderen Worten werden damit die Patientendaten 134 mit der Dauerdiagnose "chronische Erkrankung" vervollständigt. Beim nächsten Öffnen der Krankenakte durch den behandelnden Arzt kann somit das Datenverarbeitungssystem 100 direkt diese Dauerdiagnose dauerhaft im Anzeigefenster 110 darstellen.

Nachdem die mit dem Diagnoseobjekt verknüpfte medizinische Diagnose als Dauerdiagnose übernommen wurde, führt das Datenverarbeitungssystem 100 weiter einen Zugriff auf die Datenbank 142 durch. Die Datenbank 142 umfasst medizinische Medikamentenobjekte 136 und entsprechende Abfragebedingungen 138. Die Abfragebedingungen werden wiederum auf die Patientendaten 134 angewandt, wobei im Falle dessen, dass eine der Abfragebedingungen (oder auch mehrere der Abfragebedingungen) für die Patientendaten 134 erfüllt ist, eine entsprechende Benutzerabfrage auf der grafischen Benutzeroberfläche 106 ausgegeben wird. Auf dieser grafischen Benutzeroberfläche werden wiederum die medizinischen Medikamentenobjekte, beispielsweise in Form von Medikamentensubstanzen oder Präparatnamen, gegebenenfalls durch PZN-Nummern, dargestellt, woraufhin der behandelnde Arzt aus der ihm so zur Verfügung gestellten Liste eines oder mehrere Medikamente auswählen kann, welcher er zur Dauermedikation des jeweiligen Patienten verwenden will. Nach Auswahl eines oder mehrerer Medikamente werden diese daraufhin im Anzeigefenster 112 der grafischen Benutzeroberfläche 106 dauerhaft dargestellt.

Das Datenverarbeitungssystem 100 ermöglicht es einem behandelnden Arzt jedoch auch noch weiter, Diagnosen in zuverlässiger Weise zu erstellen. Beispielsweise kann hierzu das Datenverarbeitungssystem 100 wiederum auf die Datenbank 122 zugreifen, um dort Regeln unter Berechnen von Grundrisiken für medizinische Diagnosen abzurufen, wobei in der Datenbank 122 ferner die medizinischen Diagnosen mit medizinischen Symptomen 130 verknüpft abgelegt sind. Durch Anwenden der Regeln 128 auf die Patientendaten 134 und Berechnen eines Grundrisikos für eine medizinische Diagnose kann dieses Grundrisiko auf der grafischen Benutzeroberfläche 106 beispielsweise wiederum in Form eines Popups dem Arzt angezeigt werden. Das Grundrisiko wird dabei vorzugsweise zusammen mit der medizinischen Diagnose dem Arzt präsentiert. Allgemein können hier vorzugsweise der Risikowahrscheinlichkeit nach sortiert verschiedene Risiken für verschiedene medizinische Diagnosen, so vorhanden, angezeigt werden. Um dabei den Arzt nicht unnötig mit unwahrscheinlichen Grundrisiken zu verwirren, werden vorzugsweise nur Grundrisiken ab einem gewissen Schwellenwert, welcher frei skalierbar ist, angezeigt. Dies hat den weiteren Vorteil, dass systemressourcenschonend gearbeitet werden kann, da in diesem Falle nicht sämtliche irrelevanten Diagnosen im Speicher des Datenverarbeitungssystems permanent vorgehalten werden müssen.

Nach Ausgabe des einen Grundrisikos oder gegebenenfalls der mehreren Grundrisiken für medizinische Diagnosen erfolgt eine Ausgabe einer Benutzerabfrage auf der grafischen Benutzeroberfläche 106, ob für diese medizinische Diagnose eine interaktive Patientendatenanalyse durchgeführt werden soll. Wird solches vom Arzt bestätigt, erfolgt eine Ausgabe einer Symptom-Benutzerabfrage, welche der mit der medizinischen Diagnose verknüpften medizinischen Symptome 130 für eine weitere Analyse der Patientendaten 134 verwendet werden sollen. Beispielsweise werden für eine vom Arzt gewählte Diagnose verschiedene Krankheitssymptome in Form einer Liste mit Checkboxen angezeigt, wobei bei jedem aktivieren einer Checkbox, das heißt dem Bestätigen des Vorhandenseins eines Krankheitssymptoms, eine dynamische Aktualisierung der Grundrisikoberechnung für den entsprechenden Diagnosebefund erfolgt. Gegebenenfalls kann der eine Diagnosebefund auch um weitere präzisiertere Diagnosebefunde auf der grafischen Benutzeroberfläche ergänzt werden. Lautete beispielsweise zunächst eine medizinische Diagnose lediglich als "Risiko von 80% für Diabetes", so kann nun durch die zusätzliche Präzisierung auf der grafischen Benutzeroberfläche 106 ausgegeben werden, dass das Risiko für "Diabetes Typ I bei 60%" liegt und das "Risiko für Diabetes Typ II bei 40%".

Erachtet nun ein behandelnder Arzt eine der medizinischen Diagnosen als gesichert, so kann er diese in entsprechender Weise bestätigen und somit mit den Patientendaten 134 verknüpft in der Datenbank 132 ablegen.

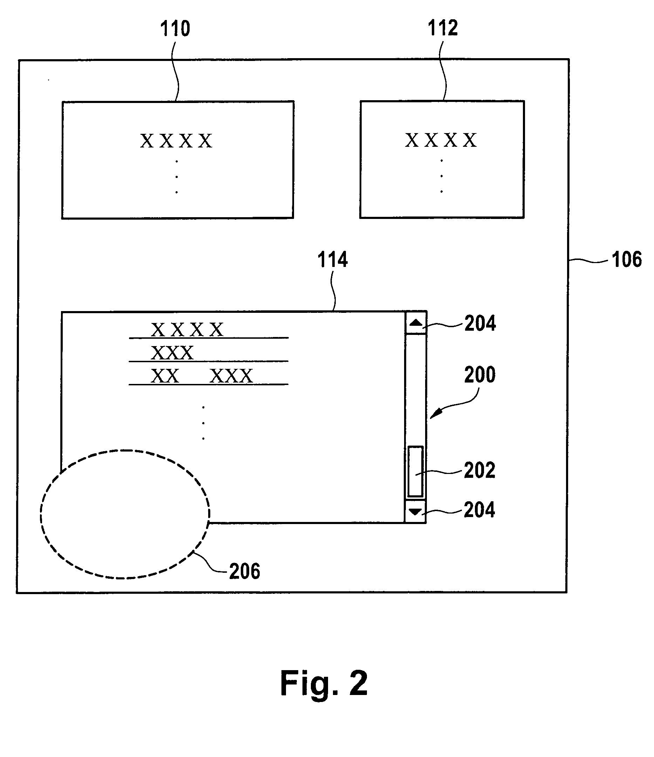

Die Figur 2 zeigt eine schematische Ansicht einer grafischen Benutzeroberfläche 106. Wie bereits hinsichtlich der Figur 1 diskutiert, weist die grafische Benutzeroberfläche 106 Anzeigefenster 110, 112 und 114 auf. Das Anzeigefenster 110 dient zur Anzeige von Dauerdiagnosen, wohingegen das Anzeigefenster 112 zur Anzeige von Dauermedikationen ausgebildet ist.

Das Anzeigefenster 114 dient zur zeilenweisen Anzeige von Patientendaten, wobei vorzugsweise beim Öffnen einer Patientenakte lediglich die wenigen, zeitlich zuletzt vorgenommenen Einträge in die Patientenakte angezeigt werden. Nichtsdestotrotz ist ein Zugriff auf weitere Einträge dadurch möglich, dass ein entsprechendes Element 202 einer Bildlaufleiste 200 (Scrollbar) vertikal hoch und runter bewegt wird, sodass durch die verschiedenen Einträge der Patientenakte geblättert werden kann. Durch Klicken auf Pfeile 204 ist es auch möglich, ein Blättern in Form von Zeilensprüngen vorzunehmen. Ferner in der Figur 2 gezeigt, ist ein Popup 206, in welchem einem Benutzer weitere Informationen zur Verfügung gestellt werden können. Bei einem solchen Popup kann es sich beispielsweise um ein Anzeigeelement mit Diagnoseobjekten, Abfragen, Medikamentenobjekten, oder aber auch Grundrisiken in Verbindung mit medizinischen Diagnosen, einem Fenster zur Durchführung einer interaktiven Patientendatenanalyse oder einem entsprechenden Abfragefenster handeln.

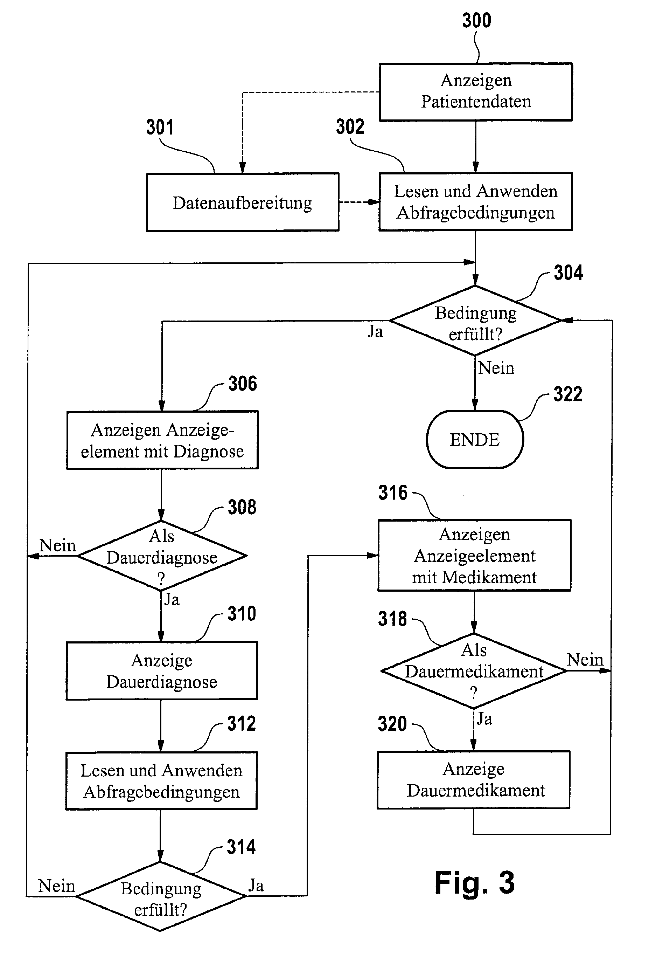

Die Figur 3 zeigt ein Flussdiagramm eines Verfahrens zur Anzeige von patientenbezogenen Diagnosen chronischer Krankheiten auf einer grafischen Benutzeroberfläche eines Datenverarbeitungssystems. Das Verfahren beginnt in Schritt 300 mit dem Anzeigen der Patientendaten in einem Anzeigefenster, wobei dieses Anzeigefenster eine Bildlaufleiste aufweist und nur ein Teil der Patientendaten in diesem Anzeigefenster angezeigt wird. In Schritt 302 erfolgt daraufhin das Lesen und Anwenden von Abfragebedingungen, wobei die Abfragebedingungen auf einer ersten Datenbank gespeichert sind, wobei die erste Datenbank medizinische Diagnoseobjekte enthält, wobei die medizinischen Diagnoseobjekte mit ersten Abfragebedingungen bezüglich der Patientendaten des Patienten verknüpft sind. Nach Schritt 302 erfolgt in Schritt 304 die Überprüfung, ob zumindest eine der ersten Abfragebedingungen für die Patientendaten erfüllt ist. Ist dies nicht der Fall, so endet das Verfahren daraufhin in Schritt 322. Ist hingegen in Schritt 304 eine der ersten Abfragebedingungen für die Patientendaten erfüllt, so setzt sich das Verfahren in Schritt 306 mit dem Anzeigen eines Anzeigeelements auf der grafischen Benutzeroberfläche fort, wobei dieses Anzeigeelement zumindest einer der Diagnoseobjekte aufweist, beispielsweise eines ICD-Codes, welches Teil des entsprechenden Diagnoseobjekts ist, für welche die erste Abfragebedingung erfüllt ist. Außerdem wird daraufhin in Schritt 308 eine Benutzerabfrage auf der grafischen Benutzeroberfläche ausgegeben, ob eine mit dem Diagnoseobjekt verknüpfte medizinische Diagnose als chronische Dauerdiagnose übernommen werden soll. Soll die medizinische Diagnose nicht als chronische Dauerdiagnose übernommen werden, so springt das Verfahren zurück zu Schritt 304, wo überprüft wird, ob eine weitere Abfragebedingung für die Patientendaten erfüllt ist. Die Schritte 304 bis 308 werden somit zyklisch für alle Abfragebedingungen durchgeführt.

Entscheidet sich in Schritt 308 der behandelnde Arzt dafür, die Diagnose als Dauerdiagnose zu übernehmen, so erfolgt in Schritt 310 die Anzeige der Dauerdiagnose beispielsweise in Form des ICD-Codes in dauerhafter Weise in einem zweiten Anzeigefenster unabhängig von der Position des Scrollbars in dem ersten Anzeigefenster.

Nach Schritt 310 beziehungsweise optional parallel zu Schritt 310 erfolgt die Ausführung von Schritt 312, dem Zugriff auf eine weitere Datenbank mit medizinischen Medikamentenobjekten, wobei die medizinischen Medikamentenobjekte mit zweiten Abfragebedingungen bezüglich der Patientendaten verknüpft sind. Die Abfragebedingungen werden in Schritt 312 gelesen und auf die Patientendaten angewandt. In Schritt 314 erfolgt die Überprüfung, ob zumindest eine der Abfragebedingungen für die Patientendaten erfüllt ist. Ist dies nicht der Fall, springt das Verfahren zurück zu Schritt 304, wo weiter in zyklischer Weise mit den Schritten 304, 306 und 308 überprüft wird, ob zumindest einer der Abfragebedingungen hinsichtlich einer chronischen Erkrankung erfüllt sind.

Ist jedoch in Schritt 314 zumindest eine der zweiten Abfragebedingungen für die Patientendaten erfüllt, so setzt sich das Verfahren in Schritt 316 mit dem Anzeigen eines Anzeigeelements auf der grafischen Benutzeroberfläche fort, sowie der Ausgabe einer Abfrage in Schritt 318, wobei das Anzeigeelement aus Schritt 316 zumindest eines der Medikamentenobjekte aufweist, für welches die zweite Abfragebedingung erfüllt ist. Hierbei kann es sich auch um einen Teil des Medikamentenobjekts, wie beispielsweise eine Pharmazentralnummer oder eine Wirkstoffbeschreibung oder einen Medikamentennamen, handeln. Die Abfrage in Schritt 318 dient dazu, um einen Arzt die Entscheidung zu ermöglichen, ob er das angezeigte Medikament zur Dauermedikation verwenden möchte. Möchte er dies nicht, springt das Verfahren zurück zu Schritt 304. Möchte er jedoch das Medikament zur Dauermedikation verwenden, so folgt dem Schritt 318 der Schritt 320 mit einer Anzeige des Medikaments in einem dritten Anzeigefenster der grafischen Benutzeroberfläche in dauerhafter Weise, das heißt unabhängig von der Position der Scrollbar. Nach Schritt 320 springt das Verfahren wiederum zu Schritt 304.

Es sei angemerkt, dass anstatt eines direkten Übergangs von Schritt 300 zu Schritt 302 auch die Möglichkeit besteht, mit einem Zwischenschritt 301 eine Datenaufbereitung der Patientendaten durchzuführen. Hierzu werden beispielsweise aus den Patientendaten jene Daten gefiltert, welche strukturiert sind. Diese strukturierten Daten werden daraufhin in einem entsprechenden Speicher, beispielsweise einem Cache-Speicher, in der Figur 1 mit dem Bezugszeichen 140 gekennzeichnet, vorgehalten.

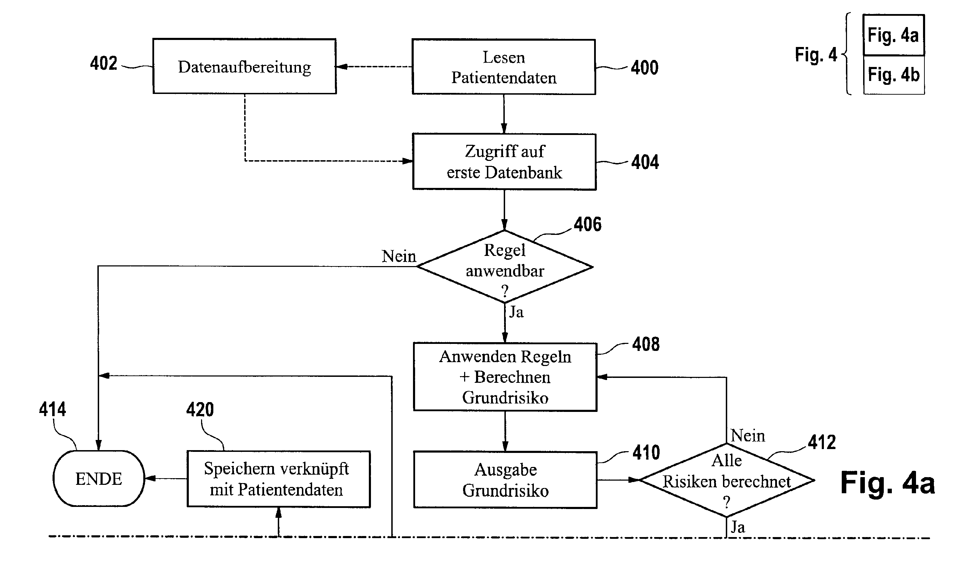

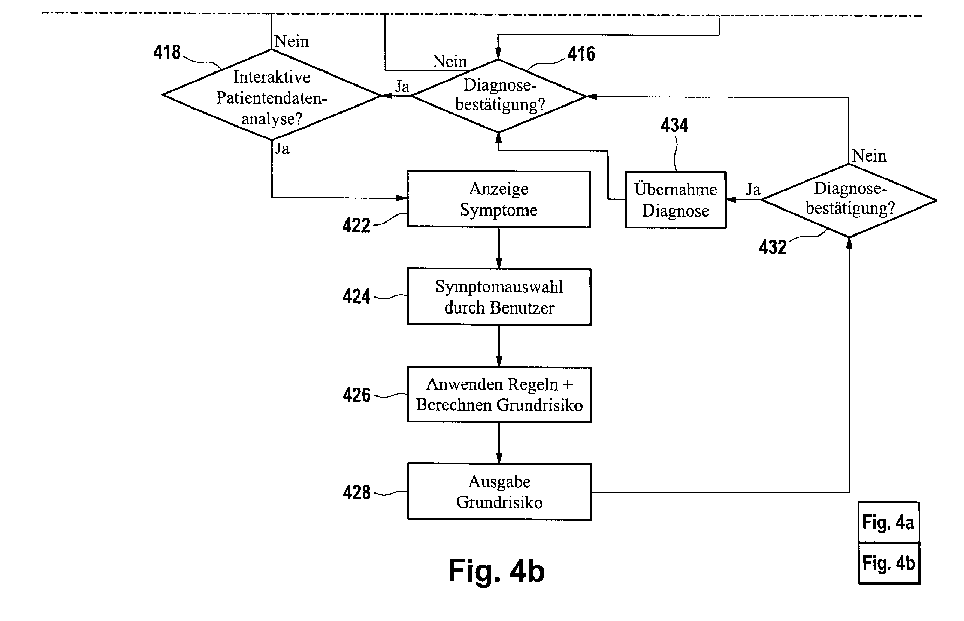

Die Figur 4 zeigt ein Flussdiagramm eines Verfahrens zur medizinischen Diagnoseunterstützung für Patientendaten eines Patienten durch ein Datenverarbeitungssystem. Das Verfahren beginnt wiederum in Schritt 400 mit dem Lesen von Patientendaten von einer entsprechenden Datenbank. Auch hier steht nach Schritt 400 wieder der optionale Schritt 402 einer Datenaufbereitung zur Verfügung, wobei entweder nach Schritt 402 oder direkt nach Schritt 400 ein Zugriff auf die erste Datenbank erfolgt, um somit Regeln zur Berechnung von Grundrisiken für medizinische Diagnosen abzurufen. In Schritt 406 erfolgt die Überprüfung, ob zumindest eine der Regeln auf die Patientendaten anwendbar ist. Ist dies nicht der Fall, weil zum Beispiel zu wenig Patientendaten vorliegen oder weil die zur Verfügung stehenden Patientendaten zu alt sind, so endet das Verfahren in Schritt 414. Ist jedoch zumindest eine der Regeln in Schritt 406 anwendbar, so erfolgt daraufhin Schritt 408, in welchem die Regeln auf die Patientendaten angewandt werden, wodurch eine Berechnung eines Grundrisikos für eine erste medizinische Diagnose erfolgt. Diese erste medizinische Diagnose wird in Schritt 410 zusammen mit dem ersten Grundrisiko auf der grafischen Benutzeroberfläche ausgegeben. Nach Schritt 410 erfolgt in Schritt 412 eine Überprüfung, ob alle Risiken für alle möglichen medizinischen Diagnosen berechnet wurden. Ist dies nicht der Fall, setzt sich das Verfahren wiederum mit den Schritten 408 und 410, gefolgt wiederum von Schritt 412, fort.

Es sei angemerkt, dass in der Figur 4 nicht die zusätzliche Möglichkeit gezeigt ist, eine Ausgabe von Grundrisiken auf eine entsprechende Mindestwahrscheinlichkeit zu beschränken, ab welcher entsprechende Grundrisiken auf der grafischen Benutzeroberfläche überhaupt erst ausgegeben werden.

Wenn sich in Schritt 412 ergibt, dass alle Risiken berechnet wurden, so setzt sich das Verfahren in Schritt 416 mit der Ausgabe einer Benutzerabfrage fort, ob die mit einem bestimmten Risiko bezeichnete Diagnose als gesicherte Diagnose in die Patientendaten übernommen werden kann. Ist dies für keines der berechneten Diagnoseergebnisse der Fall, so endet das Verfahren in Schritt 414. Es ist jedoch auch möglich, direkt eines der angezeigten Diagnoseergebnisse, beispielsweise bei einer hohen Diagnosewahrscheinlichkeit, entweder automatisch oder nach Bestätigung durch den behandelnden Arzt mit den Patientendaten verknüpft in der entsprechenden Patientendatenbank in Schritt 420 zu speichern, wobei daraufhin nach Schritt 420 das Verfahren in Schritt 414 endet. Alternativ dazu ist es möglich, im Falle einer Diagnosebestätigung in Schritt 416 dem Arzt in Schritt 418 die Möglichkeit zu bieten, eine interaktive Patientendatenanalyse durchzuführen. Möchte der Arzt eine solche Analyse nicht durchführen, erfolgt nach Schritt 418 der bereits angesprochene Schritt 420 mit dem Speichern der Diagnose als gesicherte Diagnose, verknüpft mit den Patientendaten in der Patientendatenbank. Dem wiederum folgt Schritt 414 mit der Beendigung des Verfahrens.

Möchte jedoch der Arzt eine interaktive Patientendatenanalyse in Schritt 418 durchführen, so setzt sich das Verfahren in Schritt 422 fort.

Zusammengefasst dient somit Schritt 416 dazu, dem Arzt eine Wahlmöglichkeit zu geben zwischen a) direkter Übernahme eines der Diagnoseergebnisse als gesicherte Diagnose, b) Verwerfen aller Diagnoseergebnisse oder c) Durchführung einer zusätzlichen interaktiven Patientendatenanalyse für eines oder mehrerer der Diagnoseergebnisse.

Entscheidet sich nun der Arzt für Alternative c), so wird in Schritt 422 eine Checkliste mit Symptomen ausgegeben, welche mit der in Schritt 418 gewählten medizinischen Diagnose in der ersten Datenbank verknüpft sind. Dies kann beispielsweise dadurch erfolgen, dass in Schritt 422 ein Zugriff auf die erste Datenbank erfolgt, wobei eine Abfrage der ersten Datenbank hinsichtlich möglicher Symptome für eine gegebene und gewählte medizinische Diagnose durchgeführt wird. Diese entsprechenden Symptome werden daraufhin an das Datenverarbeitungssystem übermittelt bzw. von diesem abgerufen und in Schritt 422 dem Benutzer in Form einer Checkliste angezeigt. In Schritt 424 kann nun der Benutzer eines oder mehrere der Symptome auswählen bzw. alternativ auch weitere Details zu Symptomen, beispielsweise in Form von Zahleneingaben, angeben. Lautet beispielsweise ein Symptom "hoher Blutdruck", so kann der Arzt dies dadurch präzisieren, dass er einen entsprechenden Blutdruckwert zusätzlich für dieses Symptom eingibt.

Nach jeder Auswahl bzw. Präzisierung eines der Symptome erfolgt in dynamischer Weise eine aktualisierte Berechnung des Grundrisikos für die gewählte Diagnose. Zusätzlich ist es auch möglich, weitere, zu den Symptomen passende medizinische Diagnosen mit zugehörigen Grundrisiken auszugeben, welche ebenfalls mit der Präzisierung von Symptomen ein- und ausgeblendet werden.

Dieses Anwenden der Regeln unter Berücksichtigung der Patientendaten sowie der zusätzlich präzisierten Symptome durch den Benutzer und das entsprechende erneute Berechnen des Grundrisikos erfolgt in Schritt 426. Das Ausgeben des Grundrisikos zusammen mit den zusätzlichen bestimmten medizinischen Diagnosen erfolgt in Schritt 428.

Nachdem der Arzt in dem Schritt 424 entsprechende Symptome eingegeben hat und in den Schritten 426 und 428 entsprechende präzisierte Grundrisiken für mögliche medizinische Diagnosen angezeigt wurden, erhält der Arzt in Schritt 432 die Möglichkeit einer Bestätigung einer Diagnose, welche in Schritt 428 in Verbindung mit einem Grundrisiko ausgegeben wurde. Bestätigt der Arzt in Schritt 432 keine der Diagnosen, d.h. verwirft alle vorgeschlagenen Diagnosen, so folgt Schritt 432 der Schritt 416, mit welchem dem Arzt wieder das ursprüngliche Anzeigefenster angezeigt wird, in welchem die in den Schritten 408 bis 412 berechneten Grundrisiken für verschiedene Diagnosen angezeigt werden. Bestätigt hingegen der Arzt in Schritt 432 eine der in Schritt 428 ausgegebenen Diagnosen, so erfolgt in Schritt 434 eine Übernahme dieser Diagnose, wobei diese nun in präzisierter Weise in Schritt 416 dem Arzt zusammen mit den weiteren, in den Schritten 408 bis 412 berechneten Diagnosen und deren Grundrisiken zur Auswahl für eine Speicherung verknüpft mit den Patientendaten, einer weiteren interaktiven Patientendatenanalyse oder auch einem vollständigen Verwerfen sämtlicher berechneter Grundrisiken zur Verfügung gestellt wird.

Es sei angemerkt, dass anstatt der Durchführung der Schritte 400 und 404 auch ein Zwischenschritt 402 nach Schritt 400 folgen kann, indem, wie bereits hinsichtlich Figur 3 besprochen, eine Datenaufbereitung der Patientendaten erfolgen kann.

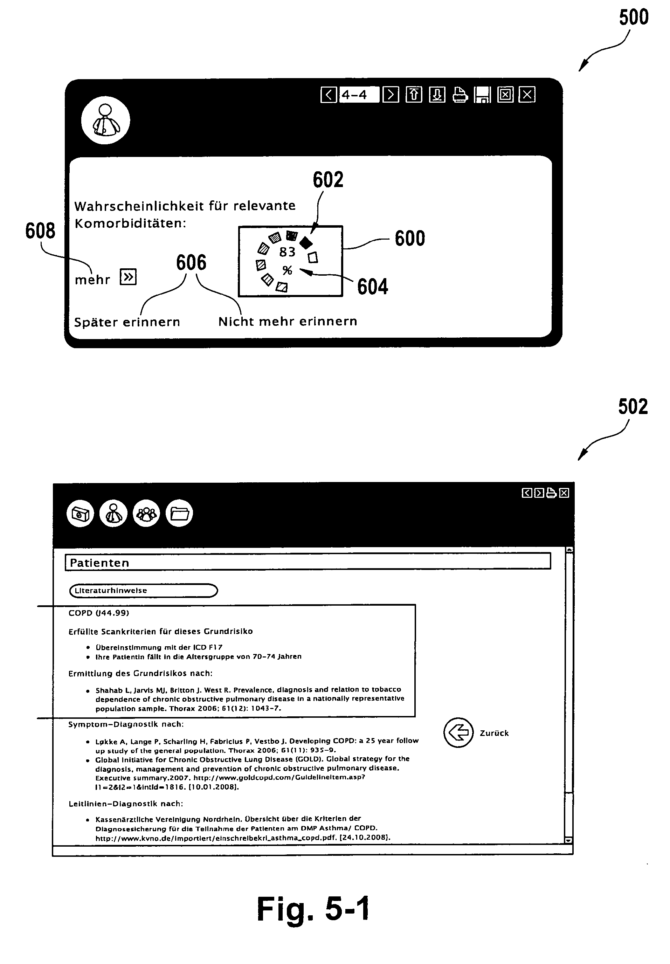

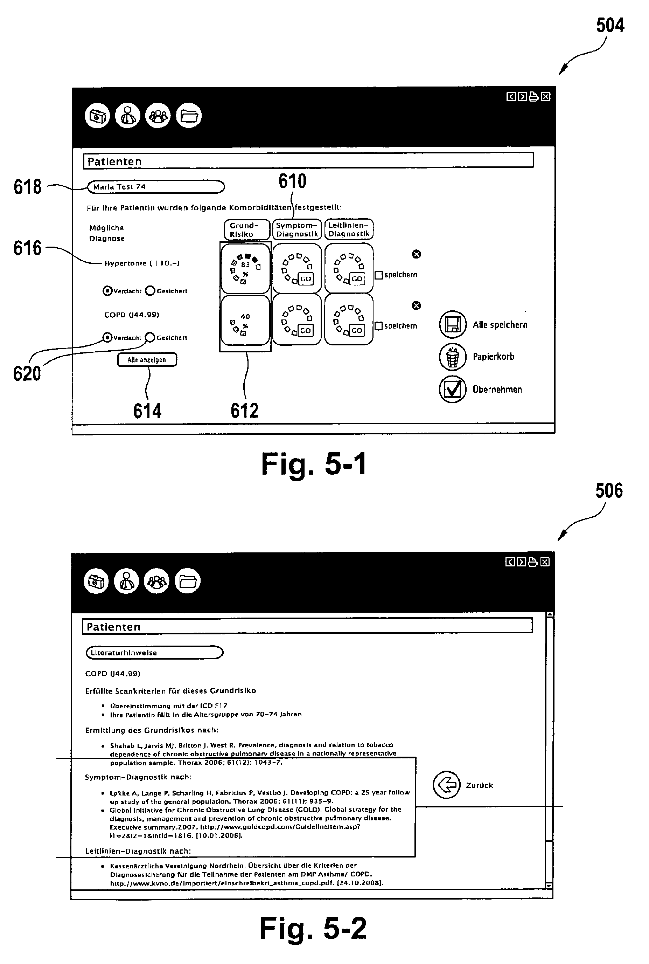

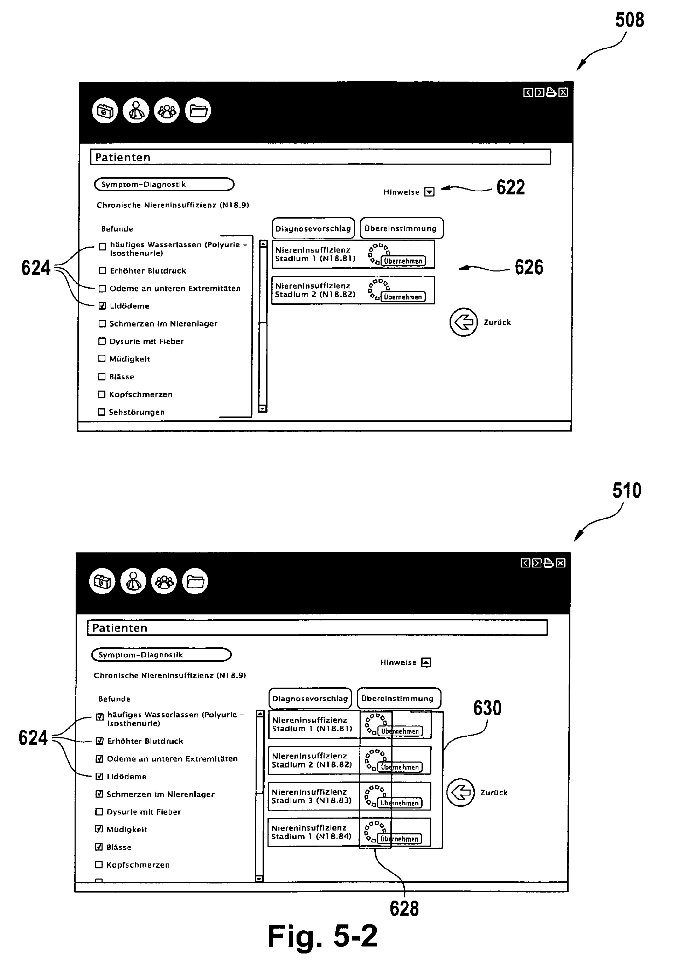

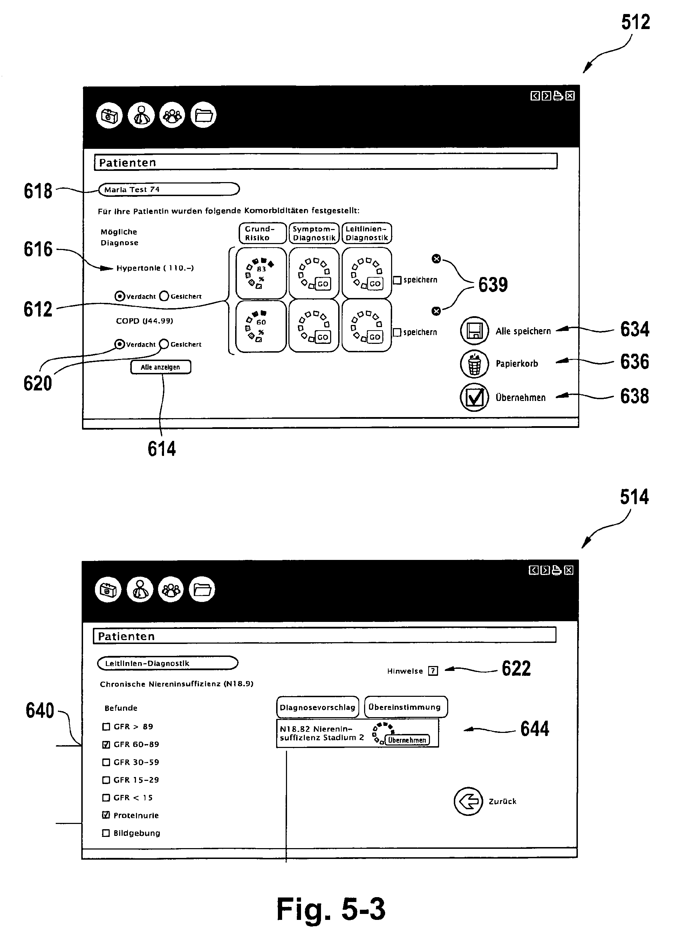

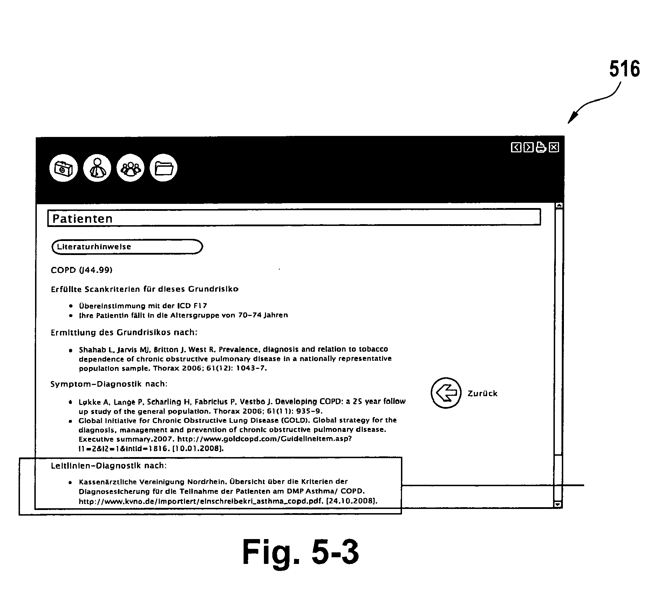

Die Figur 5 zeigt verschiedene Ausgaben auf einer grafischen Benutzeroberfläche für den Fall, dass eine medizinische Diagnoseunterstützung für Patientendaten eines Patienten durch das Datenverarbeitungssystem durchgeführt wird. Dem geht somit voraus, dass ein entsprechender Patient durch den behandelnden Arzt ausgewählt wurde und somit die Patientendaten dem Datenverarbeitungssystem zur Verfügung gestellt wurden. Daraufhin analysiert das Datenverarbeitungssystem automatisch die Patientendaten und wendet, wie hinsichtlich der Figur 4 gezeigt, Regeln auf die Patientendaten an, um zumindest ein erstes Grundrisiko für eine erste medizinische Diagnose zu berechnen.

Auf Basis des gesundheitlichen Profils des Patienten, d.h. der Patientendaten, die als strukturierte Daten in der individuellen Patientenakte auf dem Rechner des Arztes hinterlegt sind (unter anderem Alter, Geschlecht, ICD-Diagnosen, verordnete Medikamente, Laborwerte, gespeicherte Befunde und Symptome), ermittelt das Datenverarbeitungssystem die Wahrscheinlichkeit bzw. relative Häufigkeiten von relevanten Komorbiditäten bzw. häufig vergesellschafteten Krankheiten auf transparenter Leitlinien- und Literaturbasis, gleicht diese mit den schon bekannten Diagnosen ab und zeigt die bislang nicht gelisteten bzw. erkannten Erkrankungen dem Arzt patientenindividualisiert, geordnet nach Wahrscheinlichkeit, an.

Als Grundlage dient eine Liste ausgewählter medizinischer Literatur, die statistische Verbindungen zwischen den bestehenden bekannten Daten, Befunden und Erkrankungen nachgewiesen hat und nun patientenindividualisiert darstellt. Damit wird dem Arzt ein "Grundrisiko" angezeigt. Ab welchem Schwellenwert diese Anzeige greifen soll ist frei skalierbar.

Die Bildschirmausgabe 500 zeigt eine solche Ausgabe eines Grundrisikos in Form einer "Tachoscheibe" 602. Damit lassen sich Wahrscheinlichkeiten und/oder relative Häufigkeiten gleichermaßen gut visualisieren. Die Tachoscheibe besteht aus einer Skala mit farbigen Abstufungen, wobei vorzugsweise die Tachoscheibe bei hoher Wahrscheinlichkeit rote Skalenteile, bei mittlerer Wahrscheinlichkeit gelbe Skalenteile und bei niedriger Wahrscheinlichkeit grüne Skalenteile aufweist. Durch diese ampelfarbenförmige Skala ist damit ein behandelnder Arzt in rascher und einfacher Weise in der Lage, visuell die Wahrscheinlichkeit für eine relevante Komorbidität zu erfassen. Zusätzlich ist zur Präzisierung einer entsprechenden Wahrscheinlichkeit für ein Grundrisiko in der Mitte der Tachoscheibe das Grundrisiko in Form einer prozentualen Wahrscheinlichkeit 604 bzw. einer prozentualen relativen Häufigkeit 604 angegeben. Damit ist im Anzeigeelement 500 das Grundrisiko durch die Anordnung 600 in Form von Tachoscheibe und Zahlenwert gezeigt.

Ferner ist dem Arzt die Möglichkeit gegeben, die Anzeige 500 durch eine Betätigung der Schaltfläche 606 "später erinnern" für einen gewissen Zeitraum auszublenden, oder aber durch Betätigen der Schaltfläche "nicht mehr erinnern" die Anzeige 500 der Wahrscheinlichkeit für relevante Komorbiditäten vollständig auszublenden.

Das Anzeigeelement 500 dient damit dem Zweck, den Arzt in übersichtlicher und allgemeiner Weise darauf hinzuweisen, ob ein bestimmtes Grundrisiko für eine relevante medizinische Diagnose überhaupt vorliegt oder nicht. Eine Präzisierung dahingehend, wie diese medizinische Diagnose aussieht oder ob mehrere mögliche relevante medizinische Diagnosen vorliegen, ist durch das Anzeigeelement 500 nicht gegeben.

Die Kriterien für das Ermitteln einer bestimmten Wahrscheinlichkeit werden dem Arzt indikationsbezogen - auf Wunsch - transparent dargestellt, wie in Anzeigeelement 502 gezeigt. Diese Anzeige erfolgt inklusive der verwendeten Literatur- und Studienquellen als Basis des jeweiligen krankheitspezifischen Algorithmus.

Möchte nun der Arzt aufgrund der Anzeige 500 weitere Informationen hinsichtlich möglicher relevanter Komorbiditäten erhalten, so gelangt der Arzt durch Betätigen der Schaltfläche 608 "mehr" zum Anzeigeelement 504, auf welchem sich eine Zusammenfassung der für den Patienten mit dem Namen "Maria Test 74" (Bezugszeichen 618) möglichen Komorbiditäten findet. So wird im Anzeigefenster 504 die mögliche Diagnose in Form einer textuellen Beschreibung zusammen mit dem jeweiligen ICD 10 Code dargestellt (Bezugszeichen 16), zusammen mit dem jeweiligen Grundrisiko in Form einer Tachoscheibe (Bezugszeichen 612). Ferner wird dem Arzt die Möglichkeit gegeben, mittels der Auswahlelemente 620 festzulegen, ob diese angezeigten möglichen Diagnosen individuell lediglich eine Verdachtsdiagnose oder eine gesicherte Diagnose darstellen. Die Diagnose kann individuell gespeichert werden oder es können auch alle Diagnosen auf einmal gespeichert werden, d.h. in die Patientenakte übertragen werden.

Die Schaltfläche 614 "alle anzeigen" dient dazu, um weitere mögliche Diagnosen anzuzeigen, deren Grundrisiko unterhalb eines vorbestimmten Schwellwertes liegt. Im vorliegenden Fall liegt der Schwellwert beispielsweise bei 40 %, sodass hier nur mögliche Diagnosen angezeigt sind, welche ein Grundrisiko ≥ 40 % aufweisen.

Möchte der Arzt dem jeweiligen Grundrisiko nachgehen, kann der die indikationsbezogenen, jeweils literaturgestützten Leitsymptome aufrufen und mit Befunden des Patienten abgleichen lassen bzw. diese per Checkliste ergänzen. Dies geschieht, indem der Arzt in Spalte 610 auf die entsprechende Schaltfläche "GO" klickt, um somit für die jeweilige mögliche Diagnose eine Symptomdiagnostik oder Leitliniendiagnostik durchzuführen. Auch hierfür werden dem Arzt die Quellen der Symptomdiagnostik jeweils hinterlegt und transparent abgebildet, wie mit Anzeigeelement 506 illustriert. Bereits im System strukturiert hinterlegte bzw. semantisch auszuwertende Befunde werden dabei erfasst und in einer anderen farblichen Kodierung "vorausgewählt". Fährt der Arzt mit der Maus über eine so markierte Anzeige, wird ihm ein Text mit der eigenen Aktenquelle angezeigt (zum Beispiel Freitexteingabe "Konsultation vom 01.11.2008" oder "Laborwert vom 15.10.2007").

Durch Betätigen der Schaltfläche "GO" im Anzeigeelement 504 gelangt der Arzt zunächst zum Anzeigeelement 508. Das Anzeigeelement 508 weist eine Schaltfläche 622 auf, über welche der Arzt zum Anzeigeelement 506 gelangt. Außerdem weist das Anzeigeelement 622 eine Checkliste 624 mit verschiedenen Symptomen (Befunden) auf, welche symptomatisch für die mögliche Diagnose 616 sind, für welche in Anzeigeelement 504 der entsprechende Button "GO gewählt wurde. So wird in Anzeigeelement 508 für die gewählten Symptome ein Diagnosevorschlag 626 zusammen mit einer entsprechenden Übereinstimmung in Form eines neu berechneten Grundrisikos als Tachoscheibe angezeigt. Wie beispielsweise durch das Anzeigeelement 510 illustriert, erfolgt mit jeder weiteren Auswahl eines der Checkelemente eine Aktualisierung von Diagnosevorschlag und entsprechender Übereinstimmung, wodurch sich wiederum eine nach Wahrscheinlichkeiten sortierte Anordnung 628 von Grundrisiken ergibt. Wie deutlich aus dem Zusammenhang zwischen Anzeigeelement 508 und Anzeigeelement 510 entnehmbar ist, wird außerdem durch Auswahl weiterer Befunde dynamisch der zunächst gemachte Diagnosevorschlag präzisiert. So war in Anzeigeelement 508 lediglich eine Bestimmung dahingehend möglich, dass gegebenenfalls eine Niereninsuffizienz Stadium I oder Stadium II vorliegt, wohingegen in Anzeigeelement 510 aufgrund einer genaueren Präzisierung der vorliegenden Befunde eine erneute Berechnung von Grundrisiken für verschiedene medizinische Diagnosen vorgenommen werden konnte, wobei nun als zusätzliche Diagnosen eine Niereninsuffizienz Stadium III bzw. Stadium IV in Frage kommt. Außerdem wurden die Wahrscheinlichkeiten in Form der Tachoscheiben 628 in dem Anzeigeelement 510 präzisiert berechnet dargestellt.

Zusammengefasst kann mit den Anzeigeelementen 508 und 510 der behandelnde Arzt die notwendigen Symptome/Befunde ergänzen - durch Befragung des Patienten, einer Untersuchung oder das Nachtragen bereits bekannter Informationen in der Checkliste. Je nach Symptomkonstellation erscheinen dabei diejenigen Diagnosevorschläge zusammen mit ICD 10 Codes, d.h. in Klartext und Codierung, die laut angegebener Literatur, d.h. entsprechender Regeln, mit dem beschriebenen Befund korrelieren. Wieder wandelt sich die Anzeige dynamisch, d.h. der Füllungsgrad der bereits beschriebenen Tachoscheibe und Einblendung der plausiblen ICD-Diagnosen, je nach weiteren Befunden bzw. Korrelationsgrad.

Wie ferner aus Anzeigeelement 510 entnehmbar ist, kann der jeweilige Diagnosevorschlag direkt in die zentrale Übersicht übernommen werden, wobei der Vorgang mit einer oder auch mehrerer Diagnosen durchgeführt werden kann. Eine solch präzisierte zentrale Übersicht ist mit Anzeigeelement 512 dargestellt. Das Anzeigeelement 512 zeigt wiederum den Namen des Patienten 618 sowie die möglichen Diagnosen 616.

Vergleicht man das Anzeigeelement 512 mit dem Anzeigeelement 504, so ist ersichtlich, dass durch Durchführung einer Symptomdiagnostik das Grundrisiko für die Diagnose COPD (J44.99) von 40 % auf 60 % gestiegen ist.

Die zentrale Übersicht ermöglicht es nun dem Arzt, alle Komorbiditätswahrscheinlichkeiten anzeigen zu lassen (Knopf 614), und passende Diagnosen zu verwerfen (Klick auf das Kreuz 639, und gegebenenfalls später wieder reaktivieren) oder auch alle Anzeigen speichern zu lassen (Klick auf Element 634). Es können auch alle Diagnosen auf einmal verworfen werden (Klick auf Element 636) oder es können alle Diagnosen und Anzeigen durch Klick auf das Element 638 übernommen werden. Im letzteren Fall erfolgt keine Übertragung der möglichen Diagnosen und Symptome in die Patientendatenbank, sondern das System merkt sich lediglich die Ansicht 512, sodass der Arzt zu einem späteren Zeitpunkt diese Ansicht identisch wiederherstellen kann.

Eine weitere Alternative ist es, die Vorwahl "Verdacht" 620 solange bestehen zu lassen, bis eine Schwellwertwahrscheinlichkeit, welche vorzugsweise sehr hoch liegt (über 90 %) überschritten wird. Ab diesem Moment verändert sich die Wahl automatisch auf "gesichert".

Ergänzend zur automatischen Leitsymptombearbeitung kann sich der Arzt die jeweils vorgeschlagene Leitliniendiagnostik anzeigen lassen und durchlaufen, um die Diagnose abschließend zu sichern. Ein entsprechendes Anzeigefenster ist durch das Anzeigeelement 514 gegeben. Durch Anwahl des Feldes 622 wird wiederum ein Anzeigefenster 516 geöffnet, in welchem die entsprechende Leitliniendiagnostik mit entsprechenden Literaturquellen zur notwendigen bzw. empfohlenen Diagnostik sowie deren Interpretation benannt sind. Im Anzeigeelement 514 werden jeweils korrelierende Indikationen angezeigt und erneut mit einem grafischen Korrelationsgrad versehen. Die plausibelste Diagnose (oder eine andere) kann direkt in die Übersicht und in der Folge in die Akte übernommen werden.

Es sei angemerkt, dass im Falle des Vorhandenseins relevanter chronischer Erkrankungen und der Auswahl einer Dauermedikation durch den Arzt auch noch die Ausgabe eines weiteren Anzeigeelements möglich ist, welche die zeitliche Reichweite der beiden zuletzt verordneten Packungsgrößen in Relation zur vorgewählten Standarddosierung, geordnet nach Organsystem, beispielsweise parallel zur Öffnung eines Rezeptformulars, anzeigt. Hat der Arzt einen elektronischen Verordnungsplan ausgefüllt, werden diese Daten als Berechnungsgrundlage herangezogen. Darüber hinaus hat der Arzt auch die Möglichkeit, die aktuelle Dosierung direkt einzugeben und somit zeitliche Reichweitenberechnung nachzuschärfen. eine weitere Möglichkeit besteht darin, Leitliniensubstanzen nach Organsystemen vorzuschlagen, wenn eine Leitliniendiagnostik, wie in Anzeigeelement 514 der Figur 5 beschrieben, durchgeführt wird. Damit wird die Funktion der Anzeige einer zeitlichen Reichweite von Medikamentenverpackungen um die Ausprägung erweitert, dass - bei Vorlage von chronischen Erkrankungen - Leitsymptomdiagnostik zugänglich ist - bei Nichtverordnung von empfohlenen Wirkstoffen - trotz gegebener Indikation auf Literaturbasis -, nach Organsystemen geordnet, dem Arzt die empfohlenen Leitsubstanzen angezeigt werden können, um eine adäquate Versorgung des Patienten zu gewährleisten.

### Bezugszeichenliste

- 100: Datenverarbeitungssystem
- 102: Eingabemittel
- 104: Prozessor
- 106: grafische Benutzeroberfläche
- 108: Anzeigevorrichtung
- 110: Anzeigefenster
- 112: Anzeigefenster
- 114: Anzeigefenster
- 116: Speicher
- 118: Netzwerk
- 120: Schnittstelle
- 122: Datenbank
- 124: medizinisches Diagnoseobjekt
- 126: Abfragebedingung
- 128: Regeln
- 130: Symptome
- 132: Datenbank
- 134: Patientendaten
- 136: medizinisches Medikamentenobjekt
- 138: Abfragebedingung
- 140: Cache
- 142: Datenbank
- 144: Server
- 200: Scrollbar
- 202: Element
- 204: Element
- 206: Popup
- 500 - 516: Anzeigeelement
- 600: Grundrisiko
- 602: Tachoscheibe
- 604: Wahrscheinlichkeit
- 606: Eingabefläche
- 608: Eingabefläche
- 610: Symptomdiagnostik
- 612: Grundrisiko
- 616: Diagnose
- 618: Patientenname
- 620: Radio Button
- 614: Bedienelement
- 622: Bedienelement
- 624: Checkbox
- 626: Diagnosevorschlag
- 628: Grundrisiken
- 630: Schaltflächen
- 634: Schaltfläche
- 636: Schaltfläche
- 638: Schaltfläche
- 639: Schaltfläche
- 640: Befund
- 644: Diagnosevorschlag

## Patentansprüche

1. Verfahren zur Anzeige von patientenbezogenen Diagnosen chronischer Krankheiten auf einer graphischen Benutzeroberfläche (100) eines Datenverarbeitungssystems, wobei die graphische Benutzeroberfläche (100) zumindest ein erstes (114) und ein zweites (110) Anzeigefenster aufweist, mit den folgenden Schritten:
- Anzeigen von zumindest einem Teil von Patientendaten (134) eines Patienten in dem ersten Anzeigefenster, wobei die angezeigten Patientendaten in dem ersten Anzeigefenster zeilenweise angezeigt werden, wobei das erste Anzeigefenster zur zeilenweisen Nachführung der anzuzeigenden Patientendaten durch eine Scrollbar (200) ausgebildet ist,
- Zugriff auf eine erste Datenbank (122), wobei die erste Datenbank medizinische Diagnoseobjekte (124) enthält, wobei die medizinischen Diagnoseobjekte mit ersten Abfragebedingungen (126) bezüglich der Patientendaten des Patienten verknüpft sind,
- Überprüfung ob zumindest eine der ersten Abfragebedingungen für die Patientendaten erfüllt ist,
- Anzeigen eines Anzeigeelements (206) auf der graphischen Benutzeroberfläche, wenn zumindest eine der ersten Abfragebedingungen erfüllt ist, wobei das Anzeigeelement zumindest eines der Diagnoseobjekte aufweist, für welches die erste Abfragebedingung erfüllt ist,
- Ausgabe einer Benutzerabfrage auf der graphischen Benutzeroberfläche, ob eine mit dem Diagnoseobjekt verknüpfte medizinische Diagnose als chronische Dauerdiagnose übernommen werden soll,
- Dauerhaft sichtbare Anzeige der Dauerdiagnose in dem zweiten Anzeigefenster (110) unabhängig von der Position der Scrollbar (200), wenn die mit dem Diagnoseobjekt verknüpfte medizinische Diagnose als Dauerdiagnose übernommen werden soll.

2. Verfahren nach Anspruch 1, wobei die Patientendaten von einer zweiten Datenbank (132) empfangen werden, wobei das Verfahren ferner das Speichern der Dauerdiagnose in der zweiten Datenbank mit den Patientendaten verknüpft umfasst.

3. Verfahren nach einem der vorigen Ansprüche, wobei die graphische Benutzeroberfläche ferner ein drittes Anzeigefenster (112) aufweist, wobei das Verfahren, wenn die mit dem Diagnoseobjekt verknüpfte medizinische Diagnose als Dauerdiagnose übernommen werden soll, ferner umfasst:
- Zugriff auf eine dritte Datenbank (142), wobei die dritte Datenbank medizinische Medikamentenobjekte (136) enthält, wobei die medizinischen Medikamentenobjekte mit zweiten Abfragebedingungen (138) bezüglich der empfangenen Patientendaten verknüpft sind,
- Überprüfung ob zumindest eine der zweiten Abfragebedingungen für die Patientendaten erfüllt ist,
- Anzeigen eines weiteren Anzeigeelements auf der graphischen Benutzeroberfläche, wenn zumindest eine der zweiten Abfragebedingungen erfüllt ist, wobei das weitere Anzeigeelement zumindest eines der Medikamentenobjekte (136) aufweist, für welches die zweite Abfragebedingung erfüllt ist,
- Ausgabe einer weiteren Benutzerabfrage auf der graphischen Benutzeroberfläche, ob ein mit dem Medikamentenobjekt verknüpftes Medikament als Präparat zur Dauermedikation übernommen werden soll,
- Dauerhaft sichtbare Anzeige der Dauermedikation in dem dritten Anzeigefenster, unabhängig von der Position der Scrollbar, wenn das mit dem Medikamentenobjekt verknüpfte Medikament als Präparat zur Dauermedikation übernommen werden soll.

4. Verfahren nach einem der vorigen Ansprüche, wobei die Abfragebedingungen (126; 138) mit einer Zeitkonstante für ein maximales Alter verknüpft sind, wobei die Überprüfung, ob zumindest eine der ersten und/oder zweiten Abfragebedingungen erfüllt ist, nur auf die Patientendaten angewendet wird, welche einen jüngeren Zeitstempel als das maximale Alter aufweisen.

5. Verfahren nach einem der vorigen Ansprüche 2 bis 4, ferner mit dem Schritt einer Aufbereitung der Patientendaten (134), wobei die Überprüfung, ob zumindest eine der ersten und/oder zweiten Abfragebedingungen erfüllt ist, nur für die aufbereiteten Patientendaten durchgeführt wird, wobei die Datenaufbereitung die Filterung von strukturierten Daten aus den Patientendaten umfasst.

6. Verfahren nach Anspruch 5, wobei die Datenaufbereitung ferner die semantische Umwandlung von Freitextinformationen der Patientendaten in strukturierte Daten umfasst.

7. Verfahren nach einem der vorigen Ansprüche, wobei es sich bei den ersten Abfragebedingungen um Abfragen für das Vorhandensein von einer oder mehrerer in den Patientendaten enthaltenen Diagnosen handelt, wobei die medizinischen Diagnoseobjekte mit Wahrscheinlichkeiten für das Vorhandensein einer chronischen Erkrankung verknüpft sind, wobei die Diagnoseobjekte ferner mit einem Schwellwert verknüpft sind, wobei eine erste Abfragebedingung für ein Diagnoseobjekt nur dann erfüllt ist, wenn die Gesamtwahrscheinlichkeit für das Vorhandensein einer chronischen Erkrankung höher ist als der mit dem Diagnoseobjekt verknüpfte Schwellwert.

8. Verfahren nach einem der vorigen Ansprüche, wobei die Überprüfung ob zumindest eine der ersten Abfragebedingungen für die Patientendaten erfüllt ist in der Reihenfolge abnehmender Trefferwahrscheinlichkeit der Abfragebedingungen durchgeführt wird.

9. Verfahren nach einem der vorigen Ansprüche, ferner mit den folgenden Schritten:
- Zugriff auf die erste Datenbank, wobei die erste Datenbank Regeln (128) enthält, wobei die Regeln zur Berechnung von Grundrisiken für medizinische Diagnosen ausgebildet sind, wobei in der ersten Datenbank ferner die medizinischen Diagnosen mit medizinischen Symptomen (130) verknüpft gespeichert sind,
- Anwenden der Regeln auf die Patientendaten und Berechnen zumindest eines ersten Grundrisikos (612) für eine erste medizinische Diagnose (616), wenn zumindest eine der Regeln auf die Patientendaten anwendbar ist,
- Ausgabe (504) des ersten Grundrisikos (612) für die erste medizinische Diagnose zusammen mit der ersten medizinischen Diagnose (616) auf der graphischen Benutzeroberfläche,
- Ausgabe einer Benutzerabfrage (610), ob für die erste medizinische Diagnose eine interaktive Patientendatenanalyse durchgeführt werden soll,
wobei im Falle dessen, dass für die erste medizinische Diagnose eine interaktive Patientendatenanalyse durchgeführt werden soll die folgenden weiteren Schritte durchgeführt werden:
- Ausgabe einer Symptom-Benutzerabfrage (624), welche der mit der ersten medizinischen Diagnose verknüpften medizinischen Symptome für eine weitere Analyse der Patientendaten verwendet werden sollen,
- Anwenden der Regeln auf die Patientendaten und die vom Benutzer in der Symptom-Benutzerabfrage gewählten medizinischen Symptome und Berechnen zumindest eines zweiten Grundrisikos (628) für eine zweite medizinische Diagnose,
- Ausgabe (510) des zweiten Grundrisikos zusammen mit der zweiten medizinischen Diagnose auf der graphischen Benutzeroberfläche.

10. Verfahren nach Anspruch 9, wobei zusammen mit dem zweiten Grundrisiko ein zweites Bedienelement (630) bereitgestellt wird, wobei das zweite Bedienelement für eine Benutzerbestätigung ausgebildet ist, wobei im Falle einer Benutzerbestätigung über das zweite Bedienelement das zweite Grundrisiko und die zweite medizinische Diagnose als neues erstes Grundrisiko und neue erste medizinischen Diagnose auf der graphischen Benutzeroberfläche ausgegeben wird.

11. Verfahren nach einem der vorigen Ansprüche 9 oder 10, wobei nach jeder Benutzerauswahl eines weiteren medizinischen Symptoms
- Erneut die Regeln auf die Patientendaten und die bisher vom Benutzer gewählten medizinischen Symptome angewendet werden,
- ein erneutes Berechnen von zumindest einem neuen zweiten Grundrisiko für eine neue zweite medizinische Diagnose dynamisch erfolgt,
- eine aktualisierte Ausgabe (510) des erneut berechneten neuen zweiten Grundrisikos zusammen mit der neuen zweiten medizinischen Diagnose auf der graphischen Benutzeroberfläche erfolgt.
- eine aktualisierte Ausgabe der Benutzerabfrage (624) erfolgt, welche der mit der neuen zweiten medizinischen Diagnose verknüpften medizinischen Symptome für eine weitere Analyse der Patientendaten verwendet werden sollen.

12. Verfahren nach Anspruch 11, wobei mit der aktualisierten Ausgabe (510) des erneut berechneten neuen zweiten Grundrisikos eine erneute aktualisierte Ausgabe der Symptom-Benutzerabfrage erfolgt, wobei die aktualisierte Ausgabe der Symptom-Benutzerabfrage angibt, welche der mit der neuen zweiten medizinischen Diagnose verknüpften medizinischen Symptome für eine weitere Analyse der Patientendaten verwendet werden sollen, wobei zuvor vom Benutzer gewählte medizinische Symptome in der aktualisierten Ausgabe der Symptom-Benutzerabfrage beibehalten werden .

13. Datenverarbeitungssystem (100) mit einer graphischen Benutzeroberfläche (106), wobei das Datenverarbeitungssystem zur Durchführung des Verfahrens zur Anzeige von patientenbezogenen Diagnosen chronischer Krankheiten nach einem der vorigen Ansprüche ausgebildet ist.

14. Computerprogrammprodukt mit von einem Prozessor ausführbaren Instruktionen zur Durchführung der Verfahrensschritte des Verfahrens gemäß den vorigen Ansprüchen 1 bis 12.
